(19) Europäisches Patentamt
European Patent Office
Office européen des brevets

(11) Numéro de publication: **0 274 929 B1**

(12) **FASCICULE DE BREVET EUROPEEN**

(45) Date de publication de fascicule du brevet: **14.08.91**

(21) Numéro de dépôt: **87402706.3**

(22) Date de dépôt: **01.12.87**

Le dossier contient des informations techniques présentées postérieurement au dépôt de la demande et ne figurant pas dans le présent fascicule.

(51) Int. Cl.[5]: **C07D 471/04**, C07D 519/00, A61K 31/435, A61K 31/495, //(C07D471/04,221:00,221:00), (C07D519/00,495:00,471:00), (C07D519/00,487:00,471:00), (C07D519/00,498:00,471:00)

# (54) Nouveaux dérivés du pyrrole, leur préparation et les compositions pharmaceutiques qui les contiennent.

(30) Priorité: **02.12.86 FR 8616794**

(43) Date de publication de la demande: **20.07.88 Bulletin 88/29**

(45) Mention de la délivrance du brevet: **14.08.91 Bulletin 91/33**

(84) Etats contractants désignés: **AT BE CH DE ES FR GB GR IT LI LU NL SE**

(56) Documents cités:
**EP-A- 0 271 404**
**FR-A- 2 215 951**
**GB-A- 1 468 497**
**EP-A- 0 274 930**
**FR-A- 2 217 000**
**US-A- 3 898 232**

(73) Titulaire: **RHONE-POULENC SANTE**
**20, avenue Raymond Aron**
**F-92160 Antony(FR)**

(72) Inventeur: **Bourzat, Jean-Dominique**
**35, rue Santos-Dumont**
**F-75015 Paris(FR)**
Inventeur: **Capet, Marc**
**10, rue de la Galaise**
**F-94320 Thiais(FR)**
Inventeur: **Cotrel, Claude**
**17 A, avenue du Dr Arnold Netter**
**F-75012 Paris(FR)**
Inventeur: **Labaudiniere, Richard**
**11, rue du Château**
**F-94400 Vitry Sur Seine(FR)**
Inventeur: **Pitchen, Philippe**
**31, avenue de la Belle Image**
**F-94440 Marolles-en-Brie(FR)**
Inventeur: **Roussel, Gérard**
**20 ter, rue des Carrières**
**F-91450 Soisy Sur Seine(FR)**

(74) Mandataire: **Pilard, Jacques et al**
**RHONE-POULENC SANTE, Service Brevets,**
**20 Avenue Raymond Aron**
**F-92165 Antony Cédex(FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services

## Description

La présente invention concerne de nouveaux dérivés du pyrrole de formule générale :

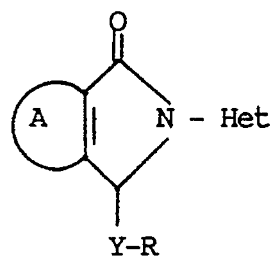

(I)

dans laquelle A forme avec le cycle pyrrole un noyau isoindoline, Y représente un atome d'oxygène ou de soufre ou un radical imino, Het représente un radical naphtyridinyle substitué par un atome d'halogène ou un radical alcoyle (1 à 4 C), alcoyloxy (1 à 4 C) et R représente un radical alcoyle non substitué ou substitué par un radical alcoylcarbonyle, dialcoylamino, alcoylcarbamoyle, dialcoylcarbamoyle, (pipérazinyl-1) carbonyle, pipéridinocarbonyle, étant entendu que les radicaux alcoyle sont en chaîne droite ou ramifiée et contiennent, sauf mention spéciale, 1 à 10 atomes de carbone et que les radicaux pipérazinyle, pipéridino peuvent être non substitués ou substitués en position quelconque par un radical alcoyle, ainsi que, lorsqu'ils existent, leurs sels pharmaceutiquement acceptables et les isomères optiques des produits de formule (I).

Dans le brevet français FR 2 217 000 et américain US 3 898 232 sont décrits des dérivés de l'isoindolinone-1 présentant des propriétés anti-arythmiques.

Dans les demandes de brevets européens EP-A-271404 et EP-A-274930 sont décrits des composés structurellement très proches des produits qui font l'objet de la présente invention et qui présentent le même type d'activité.

Selon l'invention, les produits de formule générale (I) dans laquelle Y représente un atome d'oxygène ou de soufre, Het est défini comme précédemment à l'exception de représenter un radical naphtyridinyle substitué par un radical alcoyloxy et les autres symboles sont définis comme précédemment peuvent être préparés par action d'un produit de formule générale :

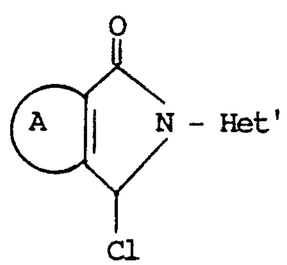

(II)

dans laquelle Het' a la définition de Het donnée précédemment à l'exception de représenter un radical naphtyridinyle substitué par un radical alcoyloxy et A est défini comme précédemment, sur un produit de formule générale :

R - Y' - H (III)

dans laquelle Y' représente un atome d'oxygène ou de soufre et R est défini comme précédemment.

On opère généralement dans un solvant organique tel qu'un éther comme le tétrahydrofuranne ou tel que le diméthylformamide en présence d'une base telle qu'un hydrure de métal alcalin comme l'hydrure de sodium, à une température comprise entre -5 et + 25 °C.

Les produits de formule générale (II) peuvent être préparés par chloruration d'un produit de formule générale :

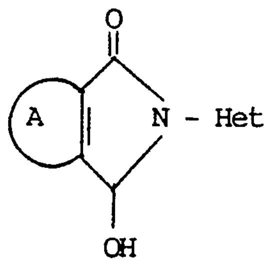

(IV)

dans laquelle A et Het sont définis comme précédemment.

On opère généralement en présence d'un agent de chloruration tel que le chlorure de sulfinyle ou l'oxychlorure de phosphore en présence de quantités catalytiques de diméthylformamide à une température comprise entre 20° C et la température de reflux du mélange réactionnel, ou de tout autre agent connu de l'homme du métier permettant de transformer un radical hydroxy en un radical chloro sans toucher au reste de la molécule.

Les produits de formule générale (IV) peuvent être préparés par application ou adaptation des méthodes décrites dans le brevet belge 835 325, 815 019 ou 808 152.

Selon l'invention, les produits de formule générale (I) peuvent également être préparés par action d'un produit de formule générale (IV) défini comme précédemment sur un dérivé de formule générale :

RX (V)

dans laquelle R est défini comme précédemment et X représente un atome d'halogène ou un reste d'ester réactif tel que mésyloxy ou tosyloxy.

On opère généralement dans un solvant organique tel que le diméthylformamide ou un carbure aromatique comme le toluène ou un mélange de ces solvants, en présence d'un hydrure alcalin comme l'hydrure de sodium à une température comprise entre 0 et 80° C.

Selon l'invention, les produits de formule générale (I) dans laquelle Y représente un atome de soufre et les autres symboles sont définis comme précédemment peuvent être préparés par action d'un produit de formule générale :

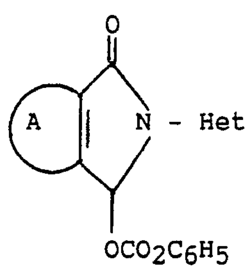

(VI)

dans laquelle A et Het sont définis comme précédemment (ou par action d'un carbonate équivalent) sur un mercaptan de formule :

R - SH (VII)

dans laquelle R est défini comme précédemment.

On opère généralement dans un solvant organique tel que l'acétonitrile à une température comprise entre 25° C et la température de reflux du mélange réactionnel.

Selon l'invention, les produits de formule générale (I) dans laquelle Y représente un radical imino et les autres symboles sont définis comme précédemment peuvent être préparés par action d'un produit de formule générale (II) ou (IV) définis comme précédemment sur une amine de formule générale :

$R - NH_2$ (VIII)

dans laquelle R est défini comme précédemment.

On opère généralement par simple chauffage à une température comprise entre 25° C et la température de reflux du mélange réactionnel dans un solvant organique tel que le tétrahydrofuranne ou le diméthylformamide.

Selon l'invention, les produits de formule générale (I) dans laquelle Y représente un atome d'oxygène, R représente un radical méthyle substitué par un radical alcoylcarbamoyle, dialcoylcarbamoyle, (pipérazinyl-1) carbonyle, pipéridinocarbonyle et les autres symboles sont définis comme précédement peuvent être préparés par action d'un composé de formule générale :

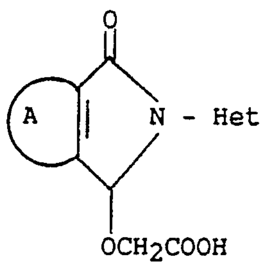

(IX)

ou une forme activée de cet acide, dans laquelle A et Het sont définis comme précédemment sur un produit de formule générale :

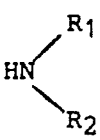

(X)

dans laquelle $R_1$ et $R_2$ représentent chacun un radical alcoyle ou bien $R_1$ représente un atome hydrogène et $R_2$ représente un radical alcoyle, ou bien $R_1$ et $R_2$ forment avec l'atome d'azote auquel ils sont liés un cycle pipérazine, pipéridine, ces cycles pouvant être substitués comme il est dit précédemment.

On opère généralement dans un solvant organique tel que le diméthylformamide en présence de diimidazolecarbonyle ou toute autre forme activée de l'acide à une température comprise entre 20 et 80° C.

L'acide de formule générale (IX) peut être obtenu par action d'un bromoacétate d'alcoyle de formule générale :

$Br - CH_2COOR_3$ (XI)

dans laquelle $R_3$ représente un radical alcoyle contenant 1 à 4 atomes de carbone en chaîne droite ou ramifiée, sur un produit de formule générale (IV) dans laquelle A et Het sont définis comme précédemment suivie d'un hydrolyse de l'ester correspondant.

La condensation du produit de formule générale (XI) sur le produit de formule générale (IV) s'effectue généralement dans un solvant organique tel que le diméthylformamide en présence d'une base telle qu'un hydrure de métal alcalin comme l'hydrure de sodium, à une température comprise entre -5 et +25° C.

L'hydrolyse de l'ester correspondant s'effectue par toute méthode connue pour transformer un ester en acide. Notamment lorsque $R_3$ représente un radical tert-butyle, il est avantageux d'effectuer l'hydrolyse au moyen de l'acide trifluoroacétique à une température voisine de 20° C.

Selon l'invention, les produits de formule générale (I) dans laquelle y représente un atome d'oxygène et les autres symboles sont définis comme précédemment peuvent être également préparés par action d'un produit de formule générale :

ROMe (XII)

dans laquelle R est défini comme précédemment et Me représente un métal alcalin tel que le sodium ou le potassium sur un produit de formule générale :

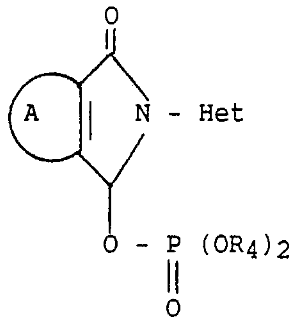

(XIII)

dans laquelle $R_4$ représente un radical alcoyle contenant 1 à 4 atomes de carbone en chaîne droite ou ramifiée ou un radical phényle et A et Het sont définis comme précédemment.

On opère généralement dans un solvant organique tel que le diméthylformamide à une température comprise entre 0° C et 25° C.

Les produits de formule générale (XIII) peuvent être préparés par action d'un produit de formule générale :

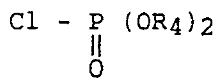

(XIV)

dans laquelle $R_4$ représente un radical alcoyle contenant 1 à 4 atomes de carbone en chaîne droite ou ramifiée ou un radical phényle sur un produit de formule générale (IV) dans laquelle A et Het sont définis comme précédemment.

On opère généralement dans un solvant organique tel que le diméthylformamide en présence d'une base telle qu'un hydrure de métal alcalin comme l'hydrure de sodium à une température comprise entre -5 et +25° C.

Il n'est pas nécessaire d'isoler le produit de formule générale (XIII) pour mettre en oeuvre le procédé selon l'invention. Il suffit d'effectuer la condensation des produits de formule générale (XIV) et (IV) comme il vient d'être dit, puis d'ajouter le produit de formule générale (XII) dans le mélange réactionnel.

Selon l'invention, les produits de formule générale (I) dans laquelle Y représente un radical imino et les autres symboles sont définis comme précédemment peuvent également être préparés par action d'un dérivé de formule générale (V) défini comme précédemment sur un produit de formule générale :

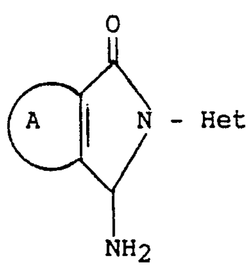

(XV)

dans laquelle A et Het sont définis comme précédemment.

On opère généralement dans un solvant chloré comme le chloroforme en présence d'un accepteur d'acide tel qu'une base comme la triéthylamine ou un carbonate ou bicarbonate alcalin, à une température comprise entre 0° C et la température de reflux du mélange réactionnel.

Les produits de formule générale (XV) peuvent être préparés par action de l'ammoniac sur un produit de formule générale (II) dans laquelle A est défini comme précédemment et Het' a la définition donnée précédemment pour Het.

Comme l'homme du métier pourra s'en rendre compte, certains radicaux entrant dans la définition du symbole R sont incompatibles avec des réactifs mis en oeuvre au cours des réactions et doivent être protégés préalablement à la mise en oeuvre des procédés ou de certaines phases des procédés exposés

précédemment. C'est notamment le cas lorsque le radical R contient des fonctions amines primaires ou secondaires ou des fonctions hydroxylées susceptibles de donner naissance à des réactions secondaires en présence d'hydrures métalliques ou de réactifs d'halogénation. Dans ce cas, les dites fonctions devront être protégées par toute méthode connue de l'homme du métier puis être débloquées après réaction.

Les nouveaux produits de formule générale (I) peuvent être purifiés par les méthodes connues habituelles, par exemple par cristallisation, chromatographie ou extractions successives en milieu acide et basique.

Les nouveaux produits de formule générale (I) peuvent être transformés en sel d'addition avec les acides par action d'un acide dans l'eau ou dans un solvant organique tel qu'un alcool, une cétone, un éther ou un solvant chloré. Le sel formé précipite, éventuellement après concentration de sa solution; il est séparé par filtration ou décantation.

Les produits de formule générale (I) présentent des propriétés pharmacologiques particulièrement intéressantes révélatrices d'une activité anxiolytique, hypnotique, anticonvulsivante, antiépileptique et myorelaxante. C'est ainsi qu'ils présentent une bonne affinité in vitro pour les sites récepteurs à benzodiazépines à des concentrations dont les valeurs sont comprises entre 0,4 et 200 nM selon la technique décrite par J.C. BLANCHARD et L. JULOU, J. of Neurochemistry, 40, 601 (1983) inspirée des travaux de SQUIRES et BRAESTRUP, Nature, 266, 732 (1977).

Chez l'animal (souris), ils se sont montrés actifs à des doses généralement comprises entre 3 et 200 mg/kg par voie orale vis-à-vis des convulsions induites par le pentétrazol selon une technique voisine de celle de EVERETT et RICHARDS, J. Pharmacol., 81, 402 (1944).

Les nouveaux produits de formule générale (I) et leurs sels présentent en outre une toxicité faible. Leur $DL_{50}$ est généralement supérieure à 300 mg/kg, par voie orale chez la souris.

Pour l'emploi médicinal, il peut être fait usage des nouveaux produits de formule générale (I) tels quels ou à l'état de sels pharmaceutiquement acceptables, c'est-à-dire non toxiques aux doses d'utilisation.

Comme exemples de sels pharmaceutiquement acceptables on peut citer les sels d'addition avec les acides minéraux tels que chlorhydrates, sulfates, nitrates, phosphates ou organiques tels que acétates, propionates, succinates, benzoates, fumarates, maléates, méthanesulfonates, iséthionates, théophyllineacétates, salicylates, phénolphtalinates, méthylène-bis-ß-oxynaphtoates ou des dérivés de substitution de ces composés.

Les exemples suivants montrent comment l'invention peut être mise en pratique.

EXEMPLE 1

A une solution de 3,1 g d'hydroxy-3 (méthoxy-7 naphtyridine-1,8 yl-2)-2 isoindolinone-1 dans 40 cm3 de diméthylformamide anhydre, maintenue sous atmosphère d'argon, on ajoute, à une température voisine de 0° C, par petites portions, 0,3 g d'hydrure de sodium. On agite la suspension obtenue pendant 45 minutes à une température voisine de 0° C puis on ajoute 8,5 cm3 d'une solution 1,4M de N,N-diméthyl chloro-2 éthylamine dans le toluène. Le mélange réactionnel est chauffé sous agitation à une température de 65° C pendant 4 heures puis est refroidi à une température voisine de 10° C, versé dans 350 cm3 d'eau distillée et extrait par 3 fois 50 cm3 de dichlorométhane. Les phases organiques sont réunies, lavées par 4 fois 30 cm3 d'eau distillée, séchées sur du sulfate de magnésium, filtrées puis concentrées à sec sous pression réduite (2,7 kPa) à 40° C. Au résidu huileux obtenu, on ajoute 8 cm3 d'éthanol chauffé à 45° C. Après élimination d'un solide par filtration, on ajoute au filtrat 1 g d'acide fumarique et chauffe jusqu'à dissolution complète. La solution obtenue est refroidie à une température voisine de 5° C pendant 1 heure. Le solide formé est séparé par filtration, lavé par 2 fois 5 cm3 d'éthanol refroidi à une température voisine de 0° C, puis par 2 fois 20 cm3 d'éther diéthylique. Au solide obtenu on ajoute 200 cm3 d'eau distillée chauffée à 40° C. Après élimination d'un solide par filtration, on refroidit le filtrat à une température voisine de 10° C puis on ajoute une solution aqueuse saturée de bicarbonate de potassium jusqu'à un pH voisin de 9 et extrait par 3 fois 120 cm3 d'acétate d'éthyle. Les phases organiques sont réunies, lavées par 3 fois 50 cm3 d'eau distillée, séchées sur du sulfate de magnésium, filtrées puis concentrées à sec sous pression réduite (2,7 kPa) à 60° C. Le résidu huileux obtenu est dissous dans 3 cm3 d'éthanol chauffé à 45° C. On ajoute à cette solution 0,4 g d'acide fumarique et chauffe jusqu'à dissolution complète. La solution obtenue est refroidie à une température voisine de 5° C pendant 1 heure. Le solide formé est séparé par filtration, lavé par 2 fois 1 cm3 d'éthanol refroidi à une température voisine de 0° C, puis par 2 fois 2 cm3 d'éther diéthylique et séché à l'air. On obtient ainsi 1,4 g de fumarate de (diméthylamino-2 éthoxy)-3 (méthoxy-7 naphtyridine-1,8 yl-2)-2 isoindolinone-1, fondant à 205° C.

L'hydroxy-3 (méthoxy-7 naphtyridine-1,8 yl-2)-2 isoindolinone-1 peut être préparée par la méthode décrite dans le brevet belge 815 019.

EXEMPLE 2

A une solution de 12,3 g d'hydroxy-3 (méthoxy-7 naphtyridine-1,8 yl-2)-2 isoindolinone-1 dans 150 cm3 de diméthylformamide anhydre, on ajoute par petites portions à une température voisine de 0 °C, 2,1 g d'une suspension huileuse (50% en poids) d'hydrure de sodium. La suspension est agitée pendant 45 minutes à cette température. On ajoute ensuite 29,4 cm3 d'une solution toluénique 1,5N de chloro-1 diméthylamino-3 propane. Le mélange est chauffé à une température voisine de 75 °C pendant 3 heures. La suspension obtenue est versée dans 900 cm3 d'eau et extraite par 3 fois 150 cm3 de chlorure de méthylène. Après lavage à l'eau et séchage sur sulfate de magnésium, les extraits organiques sont concentrés à sec sous pression réduite (2,7 kPa) à 40 °C. Le résidu obtenu est dissous dans 50 cm3 de méthanol; l'insoluble restant est éliminé par filtration. Le filtrat est concentré à sec sous pression réduite (2,7 kPa) à 70 °C. Le résidu huileux obtenu est dissous dans 20 cm3 d'éthanol. La solution obtenue est additionnée d'une solution de 2,8 g d'acide fumarique dans 35 cm3 d'éthanol. Après 2 heures à une température voisine de 4 °C, on obtient en 2 jets 4,7 g d'un solide cristallisé que l'on purifie une première fois par recristallisation dans 250 cm3 d'éthanol. On obtient ainsi 3,8 g de produit. Ce produit est repris par 200 cm3 d'eau et 100 cm3 de chlorure de méthylène, la phase aqueuse est alcalinisée à pH 9 par une solution aqueuse de soude 4N. On extrait à nouveau par 3 fois 100 cm3 de chlorure de méthylène. Après lavage à l'eau et séchage sur sulfate de magnésium, les extraits organiques sont concentrés à sec sous pression réduite (2,7 kPa) à 60 °C. L'huile obtenue (2,5 g) est dissoute dans 15 cm3 d'éthanol. A la solution obtenue, on ajoute une solution de 0.75 g d'acide fumarique dans 10 cm3 d'éthanol. Le produit cristallisé qui se forme est séparé par filtration, lavé par 5 cm3 d'éthanol et séché sous pression réduite (0,07 kPa) à 45 °C. On obtient ainsi 2,5 g de fumarate de (diméthylamino-3 propoxy)-3 (méthoxy-7 naphtyridine-1,8 yl-2)-2 isoindolinone-1, fondant à 215 - 218 °C.

EXEMPLE 3

En opérant d'une manière analogue à celle décrite à l'exemple 1, mais à partir de 15,4 g d'hydroxy-3 (méthoxy-7 naphtyridine-1,8 yl-2)-2 isoindolinone-1, de 1,5 g d'hydrure de sodium et de 32 cm3 d'une solution 0,65M de (chloro-3 propyl)-1 méthyl-4 pipérazine dans le toluène, on obtient 5,4 g de difumarate de (méthoxy-7 naphtyridine-1,8 yl-2)-2 [(méthyl-4 pipérazinyl-1)-3 propoxy]-3 isoindolinone-1, fondant à 195 °C.

La (chloro-3 propyl)-1 méthyl-4 pipérazine peut être obtenue par la méthode décrite par HROMATKA O., GRASS I., SAUTER F., Monatsh. Chem., 1956, 87, 701.

EXEMPLE 4

A une solution de 3,1 g d'hydroxy-3 (méthoxy-7 naphtyridine-1,8 yl-2)-2 isoindolinone-1 dans 50 cm3 de diméthylformamide anhydre, maintenue sous atmosphère d'argon, on ajoute, à une température voisine de 0 °C, par petites portions, 0,25 g d'hydrure de sodium. On agite la suspension obtenue pendant 45 minutes à une température voisine de 0 °C puis on ajoute 1,3 g de N,N-diméthylchloro-2 acétamide. Le mélange réactionnel est chauffé, sous agitation, à une température de 70 °C pendant 3 heures puis refroidi à une température voisine de 10 °C, versé dans 200 cm3 d'eau distillée et extrait par 3 fois 70 cm3 de dichlorométhane. Les phases organiques sont réunies, lavées par 2 fois 40 cm3 d'eau distillée, séchées sur du sulfate de magnésium, filtrées puis concentrées à sec sous pression réduite (2,7 kPa) à 40 °C. Après recristallisation dans l'acétate d'éthyle, on obtient 2 g de N,N-diméthyl [(méthoxy-7 naphtyridine-1,8 yl-2)-2 oxo-3 isoindolinyl-1] oxy-2 acétamide fondant à 166 °C.

Le chloro-2 N,N-diméthyl-acétamide peut être préparé par la méthode décrite par JACOBS W.A., HEIDELBERGER M., J. Biol. Chem. 1915, 21, 145.

EXEMPLE 5

A une solution de 3,6 g de diméthylamino-4 butanol dans 150 cm3 de tétrahydrofuranne anhydre maintenue sous atmosphère d'argon, on ajoute, à une température voisine de 0 °C, par petites portions, 1,5 g d'une suspension huileuse (50% en poids) d'hydrure de sodium. La suspension obtenue est agitée pendant 20 minutes à une température voisine de 0 °C puis refroidie à une température de -10 °C. On ajoute ensuite 9,9 g de chloro-3 (chloro-7 naphtyridine-1,8 yl-2)-2 isoindolinone-1 et poursuit l'agitation pendant 6 heures à une température voisine de 0 °C. Le mélange réactionnel est ensuite versé dans 600 cm3 d'eau distillée et extrait par 3 fois 150 cm3 de dichlorométhane. Les phases organiques sont réunies, lavées par 3 fois 40 cm3 d'eau distillée et extraites par 3 fois 100 cm3 d'une solution aqueuse d'acide

chlorhydrique 1N. Les phases aqueuses sont réunies, alcalinisées par une solution aqueuse de soude 10N jusqu'à une pH voisin de 11 puis extraites par 3 fois 200 cm3 d'acétate d'éthyle. Les phases organiques sont réunies, lavées par 4 fois 25 cm3 d'eau distillée, séchées sur du sulfate de magnésium, filtrées puis concentrées à sec sous pression réduite (2,7 kPa) à 60° C. Le résidu huileux est dissous dans 25 cm3 d'éthanol. A cette solution, on a ajoute 1,25 g d'acide fumarique puis on chauffe jusqu'à dissolution complète. La solution obtenue est refroidie à une température voisine de 5° C pendant 1 heure. Le solide formé est séparé par filtration, lavé par 2 fois 5 cm3 d'éthanol refroidi à une température voisine de 0° C, puis par 2 fois 20 cm3 d'éther diéthylique et séché à l'air. On obtient ainsi 4 g de fumarate de (chloro-7 naphtyridine-1,8 yl-)-2 (diméthylamino-4 butoxy)-3 isoindolinone-1 fondant à 190° C.

La chloro-3 (chloro-7 naphtyridine-1,8 yl-2)-2 isoindolinone-1 peut être préparée de la manière suivante : A 15,5 g d'hydroxy-3 (méthoxy-7 naphtyridine-1,8 yl-2)-2 isoindolinone-1, on ajoute goutte à goutte sous agitation 200 cm3 de chlorure de sulfinyle. Le mélange réactionnel est chauffé a reflux sous agitation pendant 1 heure puis additionné de 0,5 cm3 de diméthylformamide et chauffé à nouveau à reflux pendant 3 heures, puis refroidi à une température voisine de 60° C et concentré à sec sous pression réduite (2,7 kPa) à 60° C. Au résidu obtenu, on ajoute 100 cm3 de dichlorométhane et concentre le mélange à sec sous pression réduite (2,7 kPa) à 60° C. Au solide résiduel obtenu on ajoute 100 cm3 de dichlorométhane et agite pendant 10 minutes. Le produit est séparé par filtration et lavé par 15 cm3 de dichlorométhane puis par 2 fois 25 cm3 d'oxyde de diisopropyle et séché à l'air. On obtient ainsi 12,4 g de chloro-3 (chloro-7 naphtyridine-1,8 yl-2)-2 isoindolinone-1, infusible à 300° C.

Le diméthylamino-4 butanol peut être préparé par la méthode décrite par SZARVASI E., Bull. Soc. Chim. France, (1949), 647.

EXEMPLE 6

A une solution de 3,1 g de diméthylamino-3 propanol-1 dans 75 cm3 de tétrahydrofuranne anhydre, maintenue sous atmosphère d'argon, on ajoute, à une température voisine de 0° C, par petites portions, 1,5 g d'une suspension huileuse (50% en poids) d'hydrure de sodium et on agite la suspension obtenue pendant 30 minutes à une température voisine de 0° C. On ajoute alors 10,1 g de chloro-3 (chloro-7 naphtyridine-1,8 yl-2)-2 isoindolinone-1 et poursuit l'agitation pendant 16 heures à une température voisine de 20° C. Le mélange réactionnel est ensuite concentré à sec sous pression réduite (2,7 kPa) à 40° C. Le résidu obtenu est versé dans 200 cm3 d'eau distillée et extrait par 3 fois 150 cm3 de dichlorométhane. Les phases organiques sont réunies, lavées par 2 fois 25 cm3 d'eau distillée, séchées sur du sulfate de magnésium, filtrées puis concentrées à sec sous pression réduite (2,7 kPa) à 40° C. Le résidu huileux obtenu est dissous dans 100 cm3 de propanol-2 et la solution obtenue est additionnée de 7,65 cm3 d'une solution d'acide chlorhydrique 4N dans l'éther diéthylique. Le solide formé est séparé par filtration, lavé par 3 fois 15 cm3 de propanol-2, puis par 3 fois 25 cm3 d'oxyde de diisopropyle et séché sous pression réduite (0,07 kPa) à 40° C. Le produit ainsi obtenu est recristallisé 2 fois, d'abord dans le propanol-2 puis dans un mélange d'acétonitrile et d'oxyde de diisopropyle (60/40 en volumes). On obtient ainsi 6,3 g de chlorhydrate de (chloro-7 naphtyridine-1,8 yl-2)-2 (diméthylamino-3 propoxy)-3 isoindolinone-1, fondant à 204° C.

EXEMPLE 7

A une solution de 5,4 g d'hydroxy-3-N,N-diméthyl propanamide dans 175 cm3 de tétrahydrofuranne anhydre, maintenue sous atmosphère d'argon, on ajoute, à une température voisine de -5° C, par petites portions, 1,1 g d'hydrure de sodium et agite la suspension obtenue pendant 30 minutes une température voisine de 0° C. On ajoute alors 11,5 g de chloro-3 (chloro-7 naphtyridine-1,8 yl-2)-2 isoindolinone-1 et poursuit l'agitation pendant 3 heures à une température voisine de 20° C. Le mélange réactionnel est ensuite versé dans 600 cm3 d'eau distillée et extrait par 3 fois 200 cm3 de dichlorométhane. Les phases organiques sont réunies, lavées par 3 fois 75 cm3 d'eau distillée, séchées sur sulfate de magnésium, filtrées et concentrées à sec sous pression réduite (2,7 kPa) à 60° C. Le résidu huileux obtenu est purifié par chromatographie sur 100 g de gel de silice contenus dans une colonne de 3 cm de diamètre [éluant : dichlorométhane - méthanol (97-3 en volumes)]. On élue d'abord avec 100 cm3 de solvant : l'éluat correspondant est éliminé; on élue ensuite avec 600 cm3 de solvant : l'éluat correspondant est concentré à sec sous pression réduite (27 kPa) à 40° C. Après recristallisation dans l'acétonitrile, on obtient 4,7 g de [-(chloro-7 naphtyridine-1,8 yl-2)-2 oxo-3 isoindolinone-1] oxy-3 N,N-diméthyl-propanamide fondant à 168° C.

L'hydroxy-3 N,N-diméthyl-propanamide peut être préparé par la méthode décrite dans le brevet américain 2 548 156.

EXEMPLE 8

En opérant d'une manière analogue à celle décrite à l'exemple 7, mais à partir de 2,1 g de méthyl-4 pentanol-1, de 0,5 g d'hydrure de sodium et de 5,3 g de chloro-3 (chloro-7 naphtyridine-1,8 yl-2)-2 isoindolinone-1, on obtient 3,3 g de (chloro-7 naphtyridine-1,8 yl-2)-2 (méthyl-4 pentyloxy)-3 isoindolinone-1, fondant à 104° C.

EXEMPLE 9

En opérant d'une manière analogue à celle décrite à l'exemple 5, mais à partir de 7,35 g d'(hydroxy-3 propionyl)-1 méthyl-4 pipérazine, de 2,1 g d'une suspension huileuse (50% en poids) d'hydrure de sodium, de 11,5 g de chloro-3 (chloro-7 naphtyridine-1,8 yl-2)-2 isoindolinone-1, et de 1,75 g d'acide fumarique, on obtient 7,5 g de fumarate de (chloro-7 naphtyridine-1,8 yl-2)-2 [(méthyl-4 pipérazinyl-1) carbonyl-2 éthoxy]-3 isoindolinone-1 qu'on dissout dans 350 cm3 d'eau distillée. La solution aqueuse ainsi obtenue est alcalinisée par une solution aqueuse de soude 10N jusqu'à un pH voisin de 11, puis extraite par 3 fois 200 cm3 d'acétate d'éthyle. Les phases organiques sont réunies, lavées par 3 fois 300 cm3 d'eau distillée, séchées sur du sulfate de magnésium, filtrées puis concentrées à sec sous pression réduite (2,7 kPa) à 60° C. Après recristallisation dans l'acétate d'éthyle, on obtient 2,8 g de (chloro-7 naphtyridine-1,8 yl-2)-2 [-(méthyl-4 pipérazinyl-1) carbonyl-2 éthoxy]-3 isoindolinone-1 fondant à 150° C.

L'(hydroxy-3 propionyl)-1 méthyl-4 pipérazine peut être préparée de la manière suivante : A une solution de 7 cm3 de N-méthyl pipérazine dans 130 cm3 d'éther diéthylique anhydre, maintenue sous atmosphère d'argon, à une température voisine de -5° C, on ajoute goutte à goutte 3,15 g de $\beta$-propiolactone et agite le mélange pendant une heure à une température voisine de 20° C. Le mélange réactionnel est alors filtré et le filtrat est concentré à sec sous pression réduite (2,7 kPa) à 40° C. On obtient ainsi 8,6 g d'(hydroxy-3 propionyl)-1 méthyl-4 pipérazine sous forme d'huile utilisée à l'état brut dans les synthèses ultérieures.

EXEMPLE 10

En opérant d'une manière analogue à celle décrite à l'exemple 5, mais à partir de 4,3 g de méthyl-1 pipéridinol-4, 0,9 g d'hydrure de sodium et de 12,4 g de chloro-3 (chloro-7 naphtyridine-1,8 yl-2)-2 isoindolinone-1, et en chauffant le mélange réactionnel pendant 90 minutes à reflux, on obtient, après recristallisation dans l'éthanol 3 g de (chloro-7 naphtyridine-1,8 yl-2)-2 [(méthyl-1 pipéridyl-4) oxy]-3 isoindolinone-1 fondant à 204° C.

EXEMPLE 11

En opérant d'une manière analogue à celle décrite à l'exemple 5, mais à partir de 5,4 g de (méthyl-1 pipéridyl-4)-2 éthanol, de 1,8 g d'une suspension huileuse (50% en poids) d'hydrure de sodium, de 9,9 g de chloro-3 (chloro-7 naphtyridine-1,8 yl-2)-2 isoindolinone-1, et en agitant le mélange réactionnel pendant 2 heures à une température voisine de 20° C, on obtient, après recristallisation dans l'acétonitrile 3,65 g de (chloro-7 naphtyridine-1,8 yl-2)-2 [(méthyl-1 pipéridyl-4)-2 éthoxy]-3 isoindolinone-1, fondant à 185° C.

Le (méthyl-1 pipéridyl-4)-2 éthanol peut être préparé par la méthode décrite par KATMETANI T. et coll., 1968, 88 (5), 573. Chem. Abstr., 1969, 70, 3790p.

EXEMPLE 12

En opérant d'une manière analogue à celle décrite à l'exemple 7, mais à partir de 2,7 g de méthyl-3 butanol-1, de 0,75 g d'hydrure de sodium et de 6,6 g de chloro-3 (chloro-7 naphtyridine-1,8 yl-2)-2 isoindolinone-1, on obtient, après recristallisation dans l'oxyde de diisopropyle, 4,7 g de (chloro-7 naphtyridine-1,8 yl-2)-2 (méthyl-3 butoxy)-3 isoindolinone-1 fondant à 95° C.

EXEMPLE 13

En opérant d'une manière analogue à celle décrite à l'exemple 7, mais à partir de 3,32 g de pentanol-1, de 0,9 g d'hydrure de sodium et de 8,25 g de chloro-3 (chloro-7 naphtyridine-1,8 yl-2)-2 isoindolinone-1, on obtient, après recristallisation dans un mélange d'acétate d'éthyle et d'oxyde de diisopropyle (2-1 en volumes), 5 g de (chloro-7 naphtyridine-1,8 yl-2)-2 pentyloxy-3 isoindolinone-1, fondant à 98° C.

EXEMPLE 14

En opérant d'une manière analogue à celle décrite à l'exemple 7, mais à partir de 3,6 g de méthyl-5 hexanol-1, de 0,72 g d'hydrure de sodium, et de 6,6 g de chloro-3 (chloro-7 naphtyridine-1,8 yl-2)-2 isoindolinone-1, on obtient, après recristallisation dans l'oxyde de diisopropyle, 5,7 g de (chloro-7 naphtyridine-1,8 yl-2)-2 (méthyl-5 hexyloxy)-3 isoindolinone-1, fondant à 96° C.

Le méthyl-5 hexanol-1 peut être préparé par la méthode décrite par GRIGNARD V., C.R. Acad. Sc. Paris, 1903, 136, 1260.

EXEMPLE 15

A une solution de 6,5 g de N,N-pentaméthylène-glycolamide dans 300 cm3 de tétrahydrofuranne anhydre, maintenue sous atmosphère d'azote, on ajoute, à une température voisine de 0° C, par petites portions, 2,2 g d'une suspension huileuse (50% en poids) d'hydrure de sodium puis on ajoute 15 g de chloro-3 (chloro-7 naphtyridine-1,8 yl-2)-2 isoindolinone-1. Le mélange réactionnel est agité ensuite pendant 18 heures à une température voisine de 20° C et versé dans 1500 cm3 d'eau. Le précipité obtenu est séparé par filtration et purifié par chromatographie sur 400 g de silice (0,063-0,2 mm) contenus dans une colonne de 5 cm de diamètre [éluant : chlorure de méthylène - méthanol (95-5 en volumes)] en recueillant des fractions de 100 cm3. Les fractions 21 à 61 sont réunies et concentrées à sec sous pression réduite (2,7 kPa). Après recristallisation dans le propanol-2 puis l'acétonitrile, on obtient 7,3 g de [(chloro-7 naphtyridine-1,8 yl-2)-2 oxo-3 isoindolinyl-2] N,N-pentaméthylène-oxyacétamide fondant à 185° C.

Le N,N-pentaméthylène-glycolamide peut être obtenu de la façon suivante : Une solution de 15 g de glycolate de méthyle et de 15,8 g de pipéridine est agitée pendant 48 heures à une température voisine de 20° C. Le mélange réactionnel est ensuite concentré à sec sous pression réduite (2,7 kPa). On obtient ainsi 22,8 g de N,N-pentaméthylène-glycolamide à l'état d'huile employée brute dans les synthèses ultérieures.

EXEMPLE 16

On opère comme à l'exemple 6 mais à partir de 5,3 g d'hydroxyacétyl-1 méthyl-4 pipérazine dans 200 cm3 de tétrahydrofuranne, de 1,5 g d'une suspension huileuse (50% en poids) d'hydrure de sodium et de 10 g de chloro-3 (chloro-7 naphtyridine-1,8 yl-2)-2 isoindolinone-1. Le résidu obtenu est purifié par cristallisation dans l'acétonitrile puis par recristallisation dans le propanol-2. On obtient ainsi 3,8 g de (chloro-7 naphtyridine-1,8 yl-2)-2 [(méthyl-4 pipérazinyl-1)-2 oxo-2 éthoxy]-3 isoindolinone-1 fondant à 175° C.

L'hydroxyacétyl-1 méthyl-4 pipérazine peut être préparée selon la méthode décrite à l'exemple 15 pour la préparation du N,N-pentaméthylèneglycolamide, mais à partir de 14 g de glycolate de méthyle et de 16 g de N-méthylpipérazine. On obtient ainsi 20,2 g d'hydroxyacétyl-1 méthyl-4 pipérazine à l'état d'huile employée brute dans les synthèses ultérieures.

EXEMPLE 17

En opérant comme à l'exemple 15 mais à partir de 7 g d'hydroxyacétyl-1 méthyl-4 pipéridine dans 250 cm3 de tétrahydrofuranne, de 2,3 g d'une suspension huileuse (50% en poids) d'hydrure de sodium et de 10 g de chloro-3 (chloro-7 naphtyridine-1,8 yl-2)-2 isoindolinone-1, on obtient, après recristallisation dans le propanol-2, 2,3 g de N- (chloro-7 naphtyridine-1,8 yl-2)-2 [(méthyl-4 pipéridino)-2 oxo-2 éthoxy]-3 isoindolinone-1 fondant à 170° C.

L'hydroxyacétyl-1 méthyl-4 pipéridine peut être préparée comme à l'exemple 15 pour le N,N pentaméthylène-glycolamide, mais à partir de 14 g de glycolate de méthyle et de 15,9 g de méthyl-4 pipéridine. On obtient ainsi 21,8 g d'hydroxyacétyl-1 méthyl-4 pipéridine à l'état d'huile employée brute dans les synthèses ultérieures.

EXEMPLE 18

A une solution de 12,3 g d'hydroxy-3 (méthoxy-7 naphtyridine-1,8 yl-2)-2 isoindolinone-1 dans 150 cm3 de diméthylformamide anhydre, maintenue sous atmosphère d'azote, on ajoute, à une température voisine de 0° C, 2,1 g d'une suspension huileuse (50% en poids) d'hydrure de sodium. On agite la suspension obtenue pendant 45 minutes à une température voisine de 0° C puis on ajoute 7,7 g de N-cyclohexyl chloracétamide et chauffe le mélange à 80° C pendant 18 heures. Après refroidissement, le mélange

réactionnel est versé dans 700 cm3 d'eau et le précipité obtenu est séparé par filtration, lavé l'eau et séché à l'air. Le solide obtenu est purifié par chromatographie sur 220 g de silice (0,063-0,2 mm) contenus dans une colonne de 4 cm de diamètre (éluant : chlorure de méthylène) en recueillant des fractions de 50 cm3. Les fractions 41 à 60 sont réunies et concentrées à sec sous pression réduite (2,7 kPa). Par recristallisation du résidu obtenu dans l'acétonitrile, on obtient 1,8 g de [(méthoxy-7 naphtyridine-1,8 yl-2)-2 oxo-3 isoindolinyl-1] N-cyclohexyl-oxyacétamide fondant à 175 °C.

Le N-cyclohexyl chloracétamide peut être préparé de la façon suivante : on coule en 20 minutes, 11,3 g de chlorure de chloracétyle à une solution de 9,9 g de cyclohexylamine et de 15,2 g de triéthylamine dans 50 cm3 de chlorure de méthylène, en maintenant la température aux environs de 10 °C. Le mélange est agité ensuite pendant 2 heures à une température voisine de 20 °C puis versé dans 100 cm3 d'eau. La phase organique est séparée, lavée à l'eau et concentrée à sec sous pression réduite (2,7 kPa). On obtient ainsi 14,7 g de N-cyclohexyl-chloracétamide à l'état d'huile employée brute dans les synthèses ultérieures.

EXEMPLE 19

On opère comme à l'exemple 15 mais à partir de 9,4 g d'hydroxyacétyl-1 isopropyl-4 pipérazine dans 250 cm3 de tétrahydrofuranne, de 2,3 g d'une suspension huileuse (50% en poids) d'hydrure de sodium et de 10 g de chloro-3 (chloro-7 naphtyridine-1,8 yl-2)-2 isoindolinone-1. Après deux recristallisations successives dans l'éthanol, on obtient 2 g de (chloro-7 naphtyridine-1,8 yl-2)-2 [(isopropyl-4 pipérazino)-2 oxo-2 éthoxy]-3 isoindolinone-1 fondant à 186 °C.

L'hydroxyacétyl-1 isopropyl-4 pipérazine peut être préparée selon la méthode décrite à l'exemple 15 pour la préparation du N,N-pentaméthylèneglycolamide mais à partir de 7 g de glycolate de méthyle et de 11,1 g d'isopropyl-1 pipérazine. On obtient ainsi 15,7 g d'hydroxyacétyl-1 isopropyl-4 pipérazine à l'état d'huile employée brute dans les synthèses ultérieures.

EXEMPLE 20

A une solution de 9,3 g d'hydroxy-3 (méthoxy-7 naphtyridine-1,8 yl-2)-2 isoindolinone-1 dans 120 cm3 de diméthylformamide anhydre, maintenue sous atmosphère d'argon, on ajoute, à une température voisine de -5 °C par petites portions, 1,8 g d'une suspension huileuse (50% en poids) d'hydrure de sodium et agite la suspension obtenue pendant 30 minutes à une température voisine de 0 °C. On ajoute alors, goutte à goutte, en maintenant la température à 0 °C, 8,1 g de chloro-1 méthyl-4 pentanone-2, et poursuit l'agitation pendant encore 1 heure à 0 °C puis pendant 4 heures à une température voisine de 20 °C. Le mélange réactionnel est ensuite versé dans un mélange de 150 g de glace et 350 cm3 d'eau, neutralisé avec une solution d'acide chlorhydrique 1N et extrait par 3 fois 250 cm3 de dichlorométhane. Les phases organiques sont réunies, lavées par 4 fois 75 cm3 d'eau, séchées sur du sulfate de magnésium, filtrées puis concentrées à sec sous pression réduite (2,7 kPa) à 40 °C. Le résidu obtenu est repris par 25 cm3 d'acétate d'éthyle et le produit insoluble est séparé par filtration, lavé successivement par 10 cm3 d'acétate d'éthyle, 10 cm3 d'oxyde de diisopropyle puis l'élimine. Le filtrat est concentré à sec sous pression réduite (2,7 kPa) à 40 °C. Le résidu obtenu est purifié par chromatographie sur 120 g de silice contenus dans une colonne de 3 cm de diamètre [éluant : dichlorométhane - méthanol (98,5 - 1,5 en volumes)]. On élue d'abord avec 170 cm3 de solvant : l'éluat correspondant est éliminé; on élue ensuite avec 800 cm3 de solvant et l'éluat correspondant est concentré à sec sous pression réduite (2,7 kPa). Après recristallisation du résidu dans l'acétate d'éthyle, on obtient 3 g de (méthoxy-7 naphtyridine-1,8 yl-2)-2 (méthyl-4 oxo-2 pentyloxy)-3 isoindolinone-1 fondant à 120 °C. La chloro-1 méthyl-4 pentanone-2 peut être préparée par la méthode décrite par ASINGER F., et Coll., Ann. Chem., 1964, 672, 156.

EXEMPLE 21

On opère comme à l'exemple 15 mais à partir de 5,3 g de N-isopropyl glycolamide dans 250 cm3 de tétrahydrofuranne, de 2,2 g d'une suspension huileuse (50% en poids) d'hydrure de sodium et de 10 g de chloro-3 (chloro-7 naphtyridine-1,8 yl-2)-2 isoindolinone-1. Après purification par recristallisation dans l'acétonitrile, on obtient 4 g de [(chloro-7 naphtyridine-1,8 yl-2)-2 oxo-3 isoindolinyl-1] N-isopropyl-oxyacétamide fondant à 188 °C.

Le N-isopropylglycolamide peut être préparé selon la méthode décrite à l'exemple 15 pour la préparation du N,N-pentaméthylène-glycolamide, mais à partir de 14 g de glycolate de méthyle et de 9,5 g d'isopropylamine. On obtient ainsi 17,5 g de N-isopropylglycolamide à l'état d'huile employée brute dans les synthèses ultérieures.

EXEMPLE 22

On opère comme à l'exemple 15 mais à partir de 9,4 g de N-propyl-glycolamide dans 375 cm3 de tétrahydrofuranne, de 3,3 g d'une suspension huileuse (50% en poids) d'hydrure de sodium et de 15 g de chloro-3 (chloro-7 naphtyridine-1,8 yl-2)-2 isoindolinone-1. Après recristallisation dans l'acétonitrile, on obtient 10,5 g de [(chloro-7 naphtyridine-1,8 yl-2)-2 oxo-3 isoindolinyl-1] N-propyloxyacétamide fondant à 167° C.

Le N-propyl-glycolamide peut être préparé selon la méthode décrite à l'exemple 15 pour la préparation du N,N-pentaméthylène glycolamide mais à partir de 18 g de glycolate de méthyle et de 18 cm3 de propylamine. On obtient ainsi 22,2 g de N-propyl-glycolamide à l'état d'huile employée brute dans les synthèses ultérieures.

EXEMPLE 23

On opère comme à l'exemple 15 mais à partir de 8,9 g de N-(méthyl-2 propyl)-glycolamide dans 357 cm3 de tétrahydrofuranne, de 3,3 g d'une suspension huileuse (50% en poids) d'hydrure de sodium et de 15 g de chloro-3 (chloro-7 naphtyridine-1,8 yl-2)-2 isoindolinone-1. Après recristallisation dans l'éthanol, on obtient 10,1 g de [(chloro-7 naphtyridine-1,8 yl-2)-2 oxo-3 isoindolinyl-1] N- (méthyl-2 propyl)-oxyacétamide fondant à 146° C.

Le N-(méthyl-2 propyl)-glycolamide peut être préparé selon la méthode décrite l'exemple 15 pour la préparation du N,N-pentaméthylène-glycolamide mais à partir de 15 g de glycolate de méthyle et de 13,5 g d'isobutylamine. On obtient ainsi 21,9 g de N-(méthyl-2 propyl)-glycolamide à l'état d'huile employée brute dans les synthèses ultérieures.

EXEMPLE 24

On opère comme à l'exemple 6 mais à partir de 8 g de (diméthyl-2,6 pipéridyl-1)-4 butanol dans 220 cm3 de tétrahydrofuranne anhydre, de 1,9 g d'une suspension huileuse (50% en poids) d'hydrure de sodium et de 8,6 g de chloro-3 (chloro-7 naphtyridine-1,8 yl-2)-2 isoindolinone-1. Le résidu obtenu est repris par 20 cm3 d'acétate d'éthyle; le produit insoluble est éliminé par filtration et le filtrat est concentré à sec sous pression réduite (2,7 kPa) à 40° C. Le résidu obtenu est repris par 50 cm3 d'eau et 20 cm3 d'une solution aqueuse d'acide chlorhydrique 4N; le produit insoluble restant est éliminé par filtration. Le filtrat est neutralisé avec une solution aqueuse de soude 4N puis extrait par 3 fois 100 cm3 de chlorure de méthylène; les extraits organiques sont réunis et lavés à l'eau, séchés et concentrés à sec sous pression réduite (3 kPa) à 40° C. Le résidu obtenu est recristallisé successivement dans l'acétate d'éthyle et le propanol-2, on obtient ainsi 1,6 g de chlorhydrate de chloro-7 naphtyridine-1,8 yl-2)-2 [(diméthyl-2,6 pipéridino)-4 butoxy]-3 isoindolinone-1.

Le (diméthyl-2,6 pipéridyl-1)-4 butanol peut être préparé de la façon suivante : On ajoute en 45 minutes une solution de 15 g de (diméthyl-2,6 pipéridyl-1)-4 butyrate d'éthyle dans 50 cm3 d'éther éthylique à une suspension de 5 g d'hydrure de lithium et d'aluminium dans 250 cm3 d'éther éthylique au reflux de l'éther et agite le mélange pendant 2 heures à reflux. Après refroidissement, on ajoute en 30 minutes, 20 cm3 d'acétate d'éthyle puis successivement, 5 cm3 d'eau, 5 cm3 d'une solution aqueuse de soude 4N et 15 cm3 d'eau. Le produit insoluble est éliminé par filtration et lavé à l'éther; le filtrat est séché sur sulfate de sodium puis concentré à sec sous pression réduite (2,7 kPa) à 40° C. On obtient ainsi 8 g de (diméthyl-2,6 pipéridyl-1)-4 butanol à l'état d'huile employée brute dans les synthéses ultérieures.

Le (diméthyl-2,6 pipéridyl-1)-4 butyrate d'éthyle peut être préparé de la façon suivante : On chauffe pendant 2 heures à reflux un mélange composé de 25,8 g de bromo-3 propionate d'éthyle, 30 g de diméthyl-2,6 pipéridine et 40 cm3 d'éthanol. Le mélange réactionnel est concentré à sec sous pression réduite (2,7 kPa) et le résidu est repris par 30 cm3 d'une solution aqueuse de soude 5N puis extrait par 3 fois 50 cm3 de chlorure de méthylène. La phase organique est séchée sur sulfate de magnésium, filtrée et concentrée à sec sous pression réduite (2,7 kPa) à 40° C. On obtient ainsi 29 g de (diméthyl-2,6 pipéridyl-1)-4 butyrate d'éthyle à l'état d'huile employée brute dans les synthèses ultérieures.

EXEMPLE 25

En opérant comme à l'exemple 15 mais à partir de 9,3 g d'hydroxy-3 (méthoxy-7 naphtyridine-1,8 yl-2)-2 isoindolinone-1, de 1,8 g d'une suspension huileuse (50% en poids) d'hydrure de sodium, et de 9 g de chloro-1 méthyl-5 hexanone-2, on obtient, après recristallisation dans l'acétate d'éthyle, 3,6 g de (méthoxy-7

naphtyridine-1,8 yl-2)-2 (méthyl-5 oxo-2 hexyloxy)-3 isoindolinone-1 fondant à 120° C.

La chloro-1 méthyl-5 hexanone-2 peut être préparée par la méthode décrite par DETOEUF A., Bull. Soc. Chim. Fr., (1922), 31, 174.

EXEMPLE 26

A une solution de 7,1 g de méthyl-4 pentanethiol dans 125 cm3 de tétrahydrofuranne anhydre, maintenue sous atmosphère d'argon,on ajoute, à une température voisine de -20° C, par petites portions, 1,1 g d'hydrure de sodium et agite la suspension obtenue pendant 1 heure à une température voisine de 20° C. On ajoute alors 9,9 g de chloro-3 (chloro-7 nephtyridine-1,8 yl-2)-2 isoindolinone-1 et poursuit l'agitation du mélange réactionnel pendant 2 heures à une température voisine de 20° C. Le mélange réactionnel est additionné de 100 cm3 d'eau et de 100 cm3 d'acétate d'éthyle refroidis à une température voisine de 0° C. La phase aqueuse est séparée par décantation et extraite par 2 fois 100 cm3 d'acétate d'éthyle. Les phases organiques sont réunies, lavées par 4 fois 50 cm3 d'eau, séchées sur du sulfate de magnésium, filtrées puis concentrées à sec sous pression réduite (2,7 kPa) à 40° C. Le résidu obtenu est purifié par 6 recristallisations successives dans l'éthanol anhydre. On obtient ainsi 1,1 g de (chloro-7 nephtyridine-1,8 yl-2)-2 (méthyl-4 pentylthio)-3 isoindolinone-1 fondant à 141° C.

Le méthyl-4 pentanethiol peut être préparé par la méthode décrite par BORDWELL F.G., et HEWETT W.A., J. Org. Chem., (1957), 22, 980.

La chloro-3 (chloro-7 naphtyridine-1,8 yl-2)-2 isoindolinone-1 peut être préparée comme décrit à l'exemple 6.

EXEMPLE 27

A une suspension de 6,6 g de chloro-3 (chloro-7 naphtyridine-1,8 yl-2)-2 isoindolinone-1 dans 100 cm3 de tétrahydrofuranne anhydre, on ajoute, à une température voisine de 20° C, 4,1 g de diméthylamino-3 propylamine et chauffe le mélange réactionnel au reflux pendant 6 heures. Après avoir agité pendant encore 20 heures à une température voisine de 20° C, le mélange réactionnel est filtré. Le solide isolé est lavé par 3 fois 25 cm3 d'acétate d'éthyle. Le filtrat est lavé par 3 fois 25 cm3 d'eau distillée, séché sur du sulfate de magnésium, filtré et concentré à sec sous pression réduite (2,7 kPa) à 60° C. Au résidu obtenu, on ajoute 20 cm3 d'une solution aqueuse d'acide chlorhydrique 1N. Un produit insoluble est séparé par filtration, lavé par 2 fois 5 cm3 d'une solution aqueuse d'acide chlorhydrique 1N puis éliminé. Les filtrats aqueux sont réunis et alcalinisés par une solution aqueuse saturée de bicarbonate de potassium jusqu'à un pH voisin de 8 puis extraits par 3 fois 40 cm3 d'acétate d'éthyle. Les phases organiques sont réunies, séchées sur du sulfate de magnésium, filtrées puis concentrées à sec sous pression réduite (2,7 kPa) à 60° C. Après recristallisation dans l'acétate d'éthyle, on obtient 3 g de (chloro-7 naphtyridine-1,8 yl-2)-2 (diméthylamino-3 propylamino)-3 isoindolinone-1 fondant à 143° C.

La chloro-3 (chloro-7 naphtyridine-1,8 yl-2)-2 isoindolinone-1 peut être préparée comme décrit à l'exemple 6.

EXEMPLE 28

A une solution de 12,4 g d'hydroxy-3 (méthoxy-7 naphtyridine-1,8 yl-2)-2 isoindolinone-1, dans 200 cm3 de diméthylformamide anhydre, on ajoute, à une température voisine de 20° C, 5,1 g de diméthylamino-3 propylamine et chauffe le mélange réactionnel au reflux pendant 8 heures. Après refroidissement, le mélange réactionnel est versé dans un mélange de 1200 cm3 d'eau et de 500 cm3 d'acétate d'éthyle et filtré pour éliminer un produit insoluble. La phase aqueuse est séparée par décantation puis extraite par 2 fois 250 cm3 d'acétate d'éthyle. Les phases organiques sont réunies, lavées par 5 fois 80 cm3 d'eau, séchées sur du sulfate de magnésium, filtrées puis concentrées à sec sous pression réduite (2,7 kPa) à 60° C. Après recristallisation dans l'acétate d'éthyle, on obtient 5,1 g de (diméthylamino-3 propylamino)-3 (méthoxy-7 naphtyridine-1,8 yl-2)-2 isoindolinone-1 fondant à 128° C.

L'hydroxy-3 (méthoxy-7 naphtyridine-1,8 yl-2)-2 isoindolinone-1 peut être préparée par la méthode décrite dans le brevet belge 815 019.

EXEMPLE 29

A une solution de 11 g de carbonate mixte de (méthoxy-7 naphtyridine-1,8 yl-2)-2 oxo-3 isoindolinyle-1 et de phényle dans 130 cm3 d'acétonitrile, on ajoute 4,7 g de diméthylamino-3 propanethiol et chauffe le

mélange réactionnel au reflux pendant 6 heures. Celui-ci est ensuite concentré à sec sous pression réduite (2,7 kPa) à 60 °C et le résidu obtenu est dissous dans 600 cm3 d'acétate d'éthyle. La solution obtenue est lavée par 4 fois 40 cm3 d'eau distillée et extraite par 3 fois 150 cm3 d'une solution aqueuse d'acide chlorhydrique 1N. Les phases aqueuses sont réunies, alcalinisées par une solution aqueuse de soude 5N jusqu'à un pH voisin de 11 puis extraites par 3 fois 150 cm3 d'acétate d'éthyle. Les phases organiques sont réunies, lavées par 4 fois 40 cm3 d'eau, séchées sur du sulfate de magnésium, filtrées puis concentrées à sec sous pression réduite (2,7 kPa). Après recristallisation dans un mélange d'acétonitrile et d'oxyde de diisopropyle (50/50 en volumes), on obtient 3,8 g de (diméthylamino-3 propylthio)-3 (méthoxy-7 naphtyridine-1,8 yl-2)-2 isoindolinone-1 fondant à 113 °C.

Le carbonate de (méthoxy-7 naphtyridine-1,8 yl-2)-2 oxo-3 isoindolinyle-1 et de phényle peut être préparé de la manière suivante : A une suspension de 12,3 g d'hydroxy-3 (méthoxy-7 naphtyridine-1,8 yl-2)-2 isoindolinone-1 dans 150 cm3 de pyridine anhydre, on ajoute à une température voisine de 0 °C, 18,8 g de chloroformiate de phényle. La solution obtenue est agitée pendant 1 heure à une température voisine de 20 °C puis versée dans 500 cm3 d'eau distillée. Le produit insoluble formé est séparé par filtration et dissous dans 800 cm3 de dichlorométhane. La solution obtenue est lavée par 3 fois 75 cm3 d'eau, séchée sur du sulfate de magnésium, filtrée et concentrée à sec sous pression réduite (2,7 kPa). Après recristallisation dans l'acétonitrile, on obtient 15,6 g de carbonate de (méthoxy-7 naphtyridine-1,8 yl-2)-2 oxo-3 isoindolinyle-1 et de phényle fondant à 145 °C.

Le diméthylamino-3 propanethiol-1 peut être préparé par la méthode décrite par ANDREWS K.J.M. et coll., J. Chem. Soc., (1953), 2998 - 3002.

L'hydroxy-3 (méthoxy-7 naphtyridine-1,8 yl-2)-2 isoindolinone-1 peut être préparée par la méthode décrite dans le brevet belge 815 019.

EXEMPLE 30

A une solution de 3 g d'acide [(méthoxy-7 naphtyridine-1,8 yl-2)-2 oxo-3 isoindolinyle-1] oxyacétique dans 70 cm3 de diméthylformamide anhydre on ajoute 1,35 g de N,N'-carbonyldiimidazole et on agite pendant 2 heures à une température voisine de 25 °C. On ajoute ensuite 0,4 g de propylamine et on agite à nouveau pendant 16 heures à une température voisine de 25 °C. Le mélange réactionnel est ensuite versé dans 150 cm3 d'eau et le précipité obtenu est séparé par filtration, lavé à l'eau, séché sous pression réduite (0,05 kPa) et recristallisé dans l'éthanol. On obtient ainsi 2,95 g de [(méthoxy-7 naphtyridine-1,8 yl-2)-2 oxo-3 isoindolinyle-1] N-propyl-oxyacétamide fondant à 174 °C.

L'acide [(méthoxy-7 naphtyridine-1,8 y-2)-2 oxo-3 isoindolinyle-1] oxyacétique peut être préparé de la façon suivante : une solution de 29,4 g de [(méthoxy-7 naphtyridine-1,8 yl-2)-2 isoindolinyle-1] oxyacétate de tertiobutyle dans l'acide trifluoroacétique est agitée pendant 2 heures à une température voisine de 25 °C. Le mélange réactionnel est ensuite concentré à sec et le résidu obtenu est agité dans 200 cm3 d'oxyde d'isopropyle. Le solide cristallisé obtenu est séparé par filtration puis repris par 400 cm3 d'eau. Le mélange est amené à pH 10 avec une solution aqueuses de soude 4N. Un produit insoluble est séparé par filtration; le filtrat est lavé par 3 fois 200 cm3 d'acétate d'éthyle puis acidifié à pH 2 avec une solution aqueuse d'acide chlorhydrique 4N. Le précipité obtenu est séparé par filtration, lavé à l'eau et séché à l'air. On obtient ainsi 14,6 g d'acide [(méthoxy-7 naphtyridine-1,8 yl-2)-2 isoindolinyle-1] oxyacétique fondant à 190 °C.

Le [(méthoxy-7 naphtyridine-1,8 yl-2)-2 oxo-3 isoindolinyle-1] oxyacétate de tertiobutyle peut être préparé de la façon suivante : A une suspension de 46 g d'hydroxy-3 (méthoxy-7 naphtyridine-1,8 yl-2)-2 isoindolinone-1 dans 600 cm3 de diméthylformamide maintenue à une température voisine de 0 °C, on ajoute en 15 minutes, 7,2 g d'une suspension huileuse (50% en poids) d'hydrure de sodium et on agite pendant 1 heure et 30 minutes à cette température. On ajoute ensuite en 10 minutes une solution de 32,1 g de bromacétate de tertiobutyle dans 100 cm3 de diméthylformamide puis on agite pendant 2 heures à 60 °C. Le produit insoluble formé est séparé par filtration. Le filtrat est versé dans 300 cm3 d'eau et le produit insoluble obtenu est séparé par filtration, lavé et séché à l'air. On obtient ainsi 32,6 g de [(méthoxy-7 naphtyridine-1,8 yl-2)-2 oxo-3 isoindolinyle-1] oxyacétate de tertiobutyle fondant à 188 °C.

EXEMPLE 31

On opère comme à l'exemple 30, mais à partir de 4,5 g d'acide [(méthoxy-7 naphtyridine-1,8 yl-2)-2 oxo-3 isoindolinyle-1] oxyacétique dans 100 cm3 de diméthylformamide anhydre, de 2 g de N,N'-carbonyldiimidazole et de 0,73 g de butylamine. Après recristallisation dans l'acétonitrile, on obtient 2,35 g de [(méthoxy-7 naphtyridine-1,8 yl-2)-2 oxo-3 isoindolinyle-1] N-butyl-oxyacétamide fondant à 170 °C.

EXEMPLE 32

On opère comme à l'exemple 30, mais à partir de 3,1 g d'acide [(méthoxy-7 naphtyridine-1,8 yl-2)-2 oxo-3 isoindolinyle-1] oxyacétique dans 70 cm3 de diméthylformamide anhydre, de 1,4 g de N,N'-carbonyldiimidazole et de 0,6 g de N-méthyl propylamine. Après recristallisation dans l'éthanol, on obtient 2,7 g de [(méthoxy-7 naphtyridine-1,8 yl-2)-2 oxo-3 isoindolinyle-1] N-méthyl N-propyloxyacétamide fondant à 160° C.

EXEMPLE 33

On opère comme à l'exemple 30, mais à partir de 3 g d'acide [(méthoxy-7 naphtyridine-1,8 yl-2)-2 oxo-3 isoindolinyle-1] oxyacétique dans 65 cm3 de diméthylformamide anhydre, de 1,3 g de N,N'-carbonyldiimidazole et de 0,61 g de méthyle-3 butylamine. Après recristallisation dans l'éthanol, on obtient 2,45 g de [(méthoxy-7 naphtyridine-1,8 yl-2)-2 oxo-3 isoindolinyle-1] N-(méthyl-3 butyl)- oxyacétamide fondant à 136° C.

EXEMPLE 34

On opère comme à l'exemple 18 mais à partir de 7,3 g de (méthyl-7 naphtyridine-1,8 yl-2)- hydroxy-3 isoindolinone-1 dans 200 cm3 de diméthylformamide anhydre, de 1,8 g d'une suspension huileuse (50% en poids) d'hydrure de sodium et de 5,1 g de N-propyl-chloracétamide. Après cristallisation dans l'éther de pétrole, on obtient 1,5 g de ((méthyl-7 naphtyridine-1,8 yl-2)-2 oxo-3 isoindolinyle-1) N-propyl-glycolamide fondant à 128 - 130° C.

EXEMPLE 35

En opérant comme à l'exemple 15 mais à partir de 5,8 cm3 de butanol-1 dans 625 cm3 de diméthylformamide anhydre, de 3,1 g d'une suspension huileuse (50% en poids) d'hydrure de sodium et de 20,7 g de chloro-3 (chloro-7 naphtyridine-1,8 yl-2)-2 isoindolinone-1, on obtient après recristallisation dans l'oxyde d'isopropyle 5,6 g de butoxy-3 (chloro-7 naphtyridine-1,8 yl-2)-2 isoindolinone-1 fondant à 128° C.

EXEMPLE 36

On opère comme à l'exemple 18 mais à partir de 12 g de (bromo-7 naphtyridine-1,8 yl-2)-2 hydroxy-3 isoindolinone-1 dans 300 cm3 de diméthylformamide anhydre, de 2,4 g d'une suspension huileuse (50% en poids) d'hydrure de sodium et de 6,9 g de N-propyl chloracétamide. Après recristallisation dans l'acétonitrile, on obtient 2,1 g de [(bromo-7 naphtyridine-1,8 yl-2)-2 oxo-3 isoindolinyle-1] N-propyl-glycolamide fondant à 174° C.

Le (bromo-7 naphtyridine-1,8 yl-2)-2 hydroxy-3 isoindolinone-1 peut être préparé comme décrit dans le brevet belge 815 019.

EXEMPLE 37

A une suspension de 6,15 g d'hydroxy-3 (méthoxy-7 naphtyridine-1,8 yl-2)-2 isoindolinone-1 dans 50 cm3 de diméthylformamide anhydre, on ajoute en 15 minutes 0,96 g d'une suspension huileuse (50% en poids) d'hydrure de sodium en maintenant la température au voisinage de 0° C. On agite encore pendant 30 minutes à cette température puis on ajoute en 1 heure, 2,9 cm3 de chlorophosphate de diéthyle puis une solution de méthyl-4 pentylate de sodium préparée à partir de 2,45 g de méthyl-4 pentanol dans 30 cm3 de diméthylformamide anhydre et de 0,96 g d'une suspension huileuse (50% en poids) d'hydrure de sodium. Le mélange est ensuite agité pendant 20 heures à une température voisine de 20° C puis versé dans 400 cm3 d'eau. On extrait par 3 fois 200 cm3 de chlorure de méthylène et les phases organiques sont réunies, lavées à l'eau, séchées et concentrées à sec sous pression réduite (2,7 kPa) à 40° C. Le résidu obtenu est purifié par chromatographie sur 250 g de silice (0,063 - 0,2 mm) contenus sur une colonne de 4 cm de diamètre [éluant : acétate d'éthyle - cyclohexane (1-1 en volumes)] en recueillant des fractions de 50 cm3. Les fractions 22 à 43 sont réunies et concentrées à sec sous pression réduite (0,15 kPa). Après cristallisation du résidu dans un mélange d'oxyde de diisopropyle et d'oxyde de diéthyle (50-50 en volumes), on obtient 1,8 g de (méthoxy-7 naphtyridine-1,8 yl-2)-2 (méthyl-4 pentyloxy)-3 isoindolinone-1 fondant à 88° C.

EXEMPLE 38

A une suspension de 5 g de chloro-3 (chloro-7 naphtyridine-1,8 yl-2)-2 isoindolinone-1 dans 50 cm3 de diméthylformamide anhydre, maintenue sous atmosphère d'argon, on ajoute 2 cm3 de triéthylamine et 2 g de méthyl-4 pentylamine. Le mélange réactionnel est agité pendant 1 heure et 30 minutes à une température voisine de 20 °C, puis chauffé sous agitation, à une température de 60 °C pendant 3 heures. Après refroidissement, le mélange est versé dans 100 g de glace et 300 cm3 d'eau, puis extrait par 2 fois 200 cm3 de dichlorométhane. Les phases organiques sont réunies, lavées par 4 fois 50 cm3 d'eau, séchées sur du sulfate de magnésium, filtrées puis concentrées à sec sous pression réduite (2,7 kPa) à 40 °C. Au résidu obtenu, on ajoute 300 cm3 d'acétate d'éthyle. Un produit insoluble est éliminé par filtration et le filtrat est concentré à sec sous pression réduite (2,7 kPa) à 40 °C. Le résidu obtenu est recristallisé successivement dans l'acétate d'éthyle et l'acétonitrile. On obtient ainsi 1,4 g de (chloro-7 naphtyridine-1,8 yl-2)-2 (méthyl-4 pentylamino)-3 isoindolinone-1 fondant à 131 °C.

La méthyl-4 pentylamine peut être préparée par la méthode décrite par SHAPIRO S.L., et coll., J. Am. Chem. Soc., (1959), 81, 3728.

La présente invention concerne également les médicaments qui contiennent les produits de formule (I) à l'état pur ou sous forme de compositions dans lesquelles ils sont associés à un adjuvant, un diluant et/ou un enrobage compatible et pharmaceutiquement acceptable. Ces médicaments peuvent être employés par voie orale, rectale, parentérale ou percutanée.

Comme compositions solides pour administration orale peuvent être utilisés des comprimés, des pilules, des poudres (généralement dans des capsules de gélatine) ou des granulés. Dans ces compositions, le produit actif selon l'invention est mélangé à un ou plusieurs diluants inertes, tels que saccharose, lactose ou amidon. Ces compositions peuvent également comprendre des substances autres que les diluants, par exemple un lubrifiant tel que le stéarate de magnésium.

Comme compositions liquides pour administration orale, on peut utiliser des émulsions pharmaceutiquement acceptables, des solutions, des suspensions, des sirops et des élixirs contenant des diluants inertes tels que l'eau ou l'huile de paraffine. Ces compositions peuvent également comprendre des substances autres que les diluants, par exemple des produits mouillants, édulcorants ou aromatisants.

Les compositions selon l'invention pour administration parentérale peuvent être des solutions stériles aqueuses ou non aqueuses, des suspensions ou des émulsions. Comme solvant ou véhicule, on peut employer le propylèneglycol, un polyéthylèneglycol, des huiles végétales, en particulier l'huile d'olive ou des esters organiques injectables, par exemple l'oléate d'éthyle. Ces compositions peuvent également contenir des adjuvants, en particulier des agents mouillants, émulsifiants et dispersants. La stérilisation peut se faire de plusieurs façons, par exemple à l'aide d'un filtre bactériologique, en incorporant à la composition des agents stérilisants, par irradiation ou par chauffage. Elles peuvent également être préparées sous forme de compositions solides stériles qui peuvent être dissoutes au moment de l'emploi dans le l'eau stérile ou tout autre milieu stérile injectable.

Les compositions pour administration rectale sont des suppositoires qui peuvent contenir, outre le produit actif, des excipients tels que le beurre de cacao ou la suppo-cire.

Les compositions pour administration percutanée sont les crèmes, pommades, lotions et liniments, dans lesquels le produit actif est associé à des excipients liquides ou pâteux, de préférence en association avec un véhicule favorisant la migration percutanée.

Les médicaments et compositions selon l'invention sont particulièrement utiles en thérapeutique humaine pour leur action anxiolytique, hypnotique, anticonvulsivante, antiépileptique et myorelaxante.

En thérapeutique humaine, les doses dépendent de l'effet recherché et de la durée du traitement; elles sont généralement comprises entre 10 et 500 mg par jour par voie orale pour un adulte.

D'une façon générale, le médecin déterminera la posologie qu'il estime la plus appropriée en fonction de l'âge, du poids et de tous les autres facteurs propres au sujet à traiter.

Les exemples suivants illustrent une composition selon l'invention.

EXEMPLE A

On prépare selon la technique habituelle des comprimés dosés à 10 mg de produit actif ayant la composition suivante :

- chlorhydrate de (chloro-7 naphtyridine-1,8 yl-2)-2 (diméthylamino-3 propoxy)-3 isoindolinone-1....... 0,011 g
- amidon........................................ 0,200 g
- silice précipitée.............................. 0,035 g
- stéarate de magnésium.......................... 0,004 g

En opérant de la même manière, on peut préparer des comprimés dont le principe actif est constitué des produits suivants :

- [(chloro-7 naphtyridine-1,8 yl-2)-2 oxo-3 isoindolinyl-1] N-(méthyl-2 propyl)-oxyacétamide
- [(chloro-7 naphtyridine-1,8 yl-2)-2 oxo-3 isoindolinyl-1] N-propyloxyacétamide
- (chloro-7 naphtyridine-1,8 yl-2)-2 [(méthyl-4 pipérazinyl-1)-2 oxo-2 éthoxy]-3 isoindolinone-1
- [(chloro-7 naphtyridine-1,8 yl-2)-2 oxo-3 isoindolinyl-2] N,N-pentaméthylène-oxyacétamide
- (chloro-7 naphtyridine-1,8 yl-2)-2 (méthyl-5 hexyloxy)-3 isoindolinone-1
- [(chloro-7 naphtyridine-1,8 yl-2)-2 oxo-3 isoindolinyl-1] N-isopropyl-oxyacétamide
- [(méthoxy-7 naphtyridine-1,8 yl-2)-2 oxo-3 isoindolinyl-1] N-butyl-oxyacétamide.

**Revendications**

**Revendications pour les Etats contractants suivants : AT, BE, CH, DE, FR, GB, IT, LI, LU, NL, SE**

**1.** Nouveaux dérivés du pyrrole, caractérisés en ce qu'ils répondent à la formule générale :

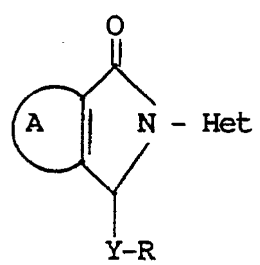

(I)

dans laquelle A forme avec le cycle pyrrole un noyau isoindoline, Y représente un atome d'oxygène ou de soufre ou un radioal imino, Het représente un radical naphtyridinyle substitué par un atome d'halogène ou un radical alcoyle (1 à 4 C), alcoyloxy (1 à 4 C), et R représente un radical alcoyle non substitué ou substitué par un radical alcoylcarbonyle, dialcoylamino, alcoylcarbamoyle, dialcoylcarbamoyle, (pipérazinyl-1) carbonyle, pipéridinocarbonyle, étant entendu que les radicaux alcoyle sont en chaîne droite ou ramifiée et contiennent, sauf mention spéciale, 1 à 10 atomes de carbone et que les radicaux pipérazinyle, pipéridino peuvent être non substitués ou substitués en position quelconque par un radical alcoyle, ainsi que, lorsqu'ils existent, leurs sels pharmaceutiquement acceptables et les isomères optiques des produits de formule (I).

**2.** Procédé de préparation d'un produit selon la revendication 1 dans la formule duquel Y représente un atome d'oxygène ou de soufre, Het est défini comme à la revendication 1 à l'exception de représenter un radical naphtyridinyle substitué par un radical alcoyloxy et les autres symboles sont définis comme à la revendication 1, caractérisé en ce que l'on fait réagir un produit de formule générale :

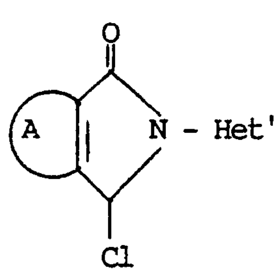

(II)

dans laquelle Het' a la définition de Het donnée à la revendication 1 à l'exception de représenter un radical naphtyridinyle substitué par un radical alcoyloxy et A est défini comme à la revendication 1, sur un produit de formule générale :

R - Y'- H (III)

dans laquelle Y' représente un atome de soufre ou d'oxygène et R est défini comme à la revendication 1, puis isole le produit obtenu et le transforme si on le désire, le cas échéant, en un sel pharmaceutiquement acceptable.

3. Procédé de préparation d'un produit selon la revendication (I), caractérisé en ce que l'on fait réagir un produit de formule générale :

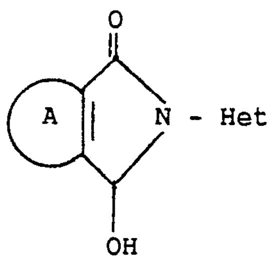

(IV)

dans laquelle Het est défini comme à la revendication 1 sur un produit de formule générale :

R - X (V)

dans laquelle R est défini comme à la revendication 1, et X représente un atome d'halogène ou un reste d'ester réactif, puis isole le produit obtenu et le transforme éventuellement, le cas échéant, en un sel pharmaceutiquement acceptable.

4. Procédé de préparation d'un produit selon la revendication 1 dans la formule duquel Y représente un atome de soufre et les autres symboles sont définis comme à la revendication 1, caractérisé en ce que l'on fait réagir un produit de formule générale :

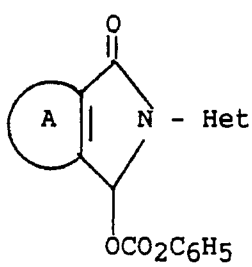

(VI)

dans laquelle A et Het sont définis comme à la revendication 1, ou un carbonate équivalent, sur un mercaptan de formule générale :

R - SH (VII)

dans laquelle R est défini comme à la revendication 1, puis isole le produit obtenu et le transforme si on le désire, le cas échéant, en un sel pharmaceutiquement acceptable.

5. Procédé de préparation d'un produit selon la revendication 1, dans la formule duquel Y représente un radical imino et les autres symboles sont définis comme à la revendication 1, caractérisé en ce que l'on fait réagir un produit de formule générale :

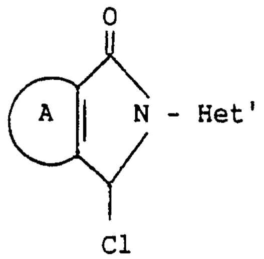 (II) ou 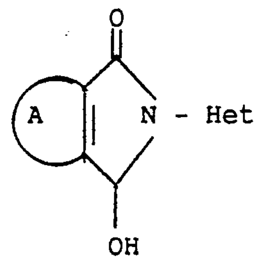 (IV)

dans lesquels A et Het sont définis comme à la revendication 1, et Het' est défini comme Het à la revendication 1 à l'exception de représenter un radical naphtyridinyle substitué par un radical alcoyloxy, sur une amine de formule générale :

$R - NH_2$ (VIII)

dans laquelle R est défini comme à la revendication 1 puis isole le produit obtenu et le transforme, si on le désire et le cas échéant, en un sel pharmaceutiquement acceptable.

**6.** Procédé de préparation d'un produit selon la revendication 1 dans la formule duquel Y représente un atome d'oxygène, R représente un radical méthyle substitué par un radical alcoylcarbamoyle, dialcoylcarbamoyle, (pipérazinyl-1) carbonyle, pipéridinocarbonyle et les autres symboles sont définis comme à la revendication 1, caractérisé en ce que l'on fait agir un composé de formule générale :

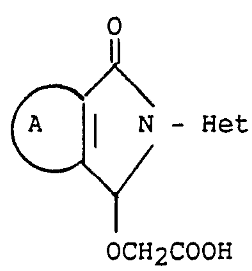 (IX)

dans laquelle A et Het sont définis comme à la revendication 1 sur un produit de formule générale :

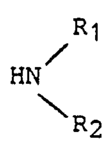 (X)

dans laquelle $R_1$ et $R_2$ représentent chacun un radical alcoyle ou bien $R_1$ représente un atome d'hydrogène et $R_2$ représente un radical alcoyle ou bien $R_1$ et $R_2$ forment avec l'atome d'azote auquel ils sont liés le cycle pipérazine ou pipéridine, ces cycles pouvant être substitués comme il est dit à la revendication 1, puis isole le produit obtenu et le transforme, si on le désire, le cas échéant, en un sel pharmaceutiquement acceptable.

**7.** Procédé de préparation d'un produit selon la revendication 1 dans la formule duquel Y représente un atome d'oxygène et les autres symboles sont définis comme à la revendication 1, caractérisé en ce que l'on fait agir un produit de formule générale :

ROMe (XII)

dans laquelle R est défini comme à la revendication 1 et Me représente un métal alcalin sur un produit de formule générale :

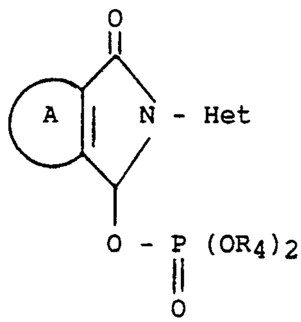

(XIII)

dans laquelle $R_4$ représente un radical alcoyle contenant 1 à 4 atomes de carbone en chaîne droite ou ramifiée ou phényle et A et Het sont définis comme à la revendication 1, puis isole le produit obtenu et le transforme, si on le désire, le cas échéant, en un sel pharmaceutiquement acceptable.

**8.** Procédé de préparation d'un produit selon la revendication 1 dans la formule duquel Y représente un radical imino et les autres symboles sont définis comme à la revendication 1, caractérisé en ce que l'on fait réagir un produit de formule générale :

R - X (V)

dans laquelle R est défini comme à la revendication 1 et X représente un atome d'halogène ou un reste d'ester réactif, sur un produit de formule générale :

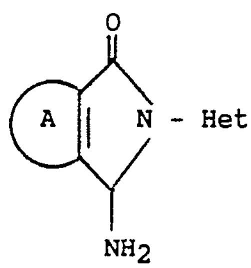

(XV)

dans laquelle A et Het sont définis comme à la revendication 1, puis isole le produit obtenu et le transforme, si on le désire, le cas échéant, en un sel pharmaceutiquement acceptable.

**9.** Compositions pharmaceutiques, caractérisées en ce qu'elles contiennent au moins un produit selon la revendication 1, en association avec un ou plusieurs diluants ou adjuvants compatibles et pharmaceutiquement acceptables.

**Revendication pour les Etats contractants suivants : ES, GR**

**1.** Procédé de préparation de nouveaux dérivés du pyrrole, de formule générale :

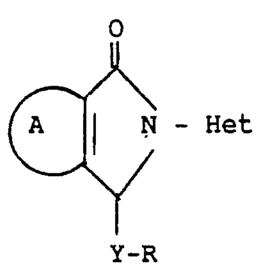

(I)

dans laquelle A forme avec le cycle pyrrole un noyau isoindoline, Y représente un atome d'oxygène ou de soufre ou un radical imino, Het représente un radical naphtyridinyle substitué par un atome d'halogène ou un radical alcoyle (1 à 4 C), alcoyloxy (1 à 4 C) et R représente un radical alcoyle non substitué ou substitué par un radical alcoylcarbonyle, dialcoylamino, alcoylcarbamoyle, dialcoylcarbamoyle, (pipérazinyl-1) carbonyle, pipéridinocarbonyle, étant entendu que les radicaux alcoyle sont en chaîne droite ou ramifiée et contiennent, sauf mention spéciale, 1 à 10 atomes de carbone et que les radicaux pipérazinyle, pipéridino peuvent être non substitués ou substitués en position quelconque par un radical alcoyle, ainsi que, lorsqu'ils existent, leurs sels pharmaceutiquement acceptables et les isomères optiques des produits de formule (I), caractérisé en ce que

A - Pour la préparation d'un produit de formule générale (I) dans laquelle Y représente un atome d'oxygène ou de soufre, Het est défini comme précédemment à l'exception de représenter un radical naphtyridinyle substitué par un radical alcoyloxy et les autres symboles sont définis comme précédemment, on fait réagir un produit de formule générale :

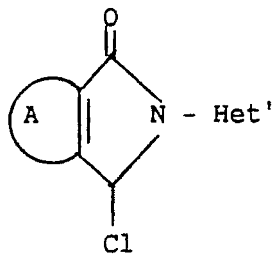

(II)

dans laquelle Het' a la définition de Het donnée précédemment à l'exception de représenter un radical naphtyridinyle substitué par un radical alcoyloxy et A est défini comme précédemment, sur un produit de formule générale :

R - Y'- H (III)

dans laquelle Y' représente un atome de soufre ou d'oxygène et R est défini comme précédemment, puis isole le produit obtenu et le transforme si on le désire, le cas échéant, en un sel pharmaceutiquement acceptable, ou en ce que

B - Pour la préparation d'un produit de formule générale (I) défini comme précédemment, on fait réagir un produit de formule générale :

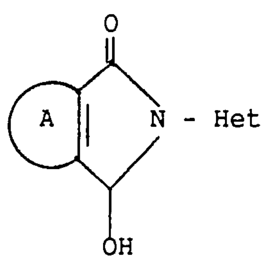

(IV)

dans laquelle Het est défini comme précédemment sur un produit de formule générale :

R - X (V)

dans laquelle R est défini comme précédemment, et X représente un atome d'halogène ou un reste d'ester réactif, puis isole le produit obtenu et le transforme éventuellement, le cas échéant, en un sel pharmaceutiquement acceptable, ou en ce que,

C - Pour la préparation d'un produit de formule générale (I) dans laquelle Y représente un atome de soufre et les autres symboles sont définis comme précédemment, on fait réagir un produit de formule générale :

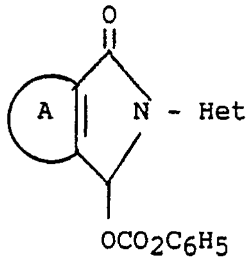

(VI)

dans laquelle A et Net sont définis comme précédemment, ou un carbonate équivalent, sur un mercaptan de formule générale :

R - SH (VII)

dans laquelle R est défini comme précédemment, puis isole le produit obtenu et le transforme si on le désire, le cas échéant, en un sel pharmaceutiquement acceptable,

D - Pour la préparation d'un produit de formule générale (I) dans laquelle Y représente un radical imino et les autres symboles sont définis comme précédemment, on fait réagir un produit de formule générale :

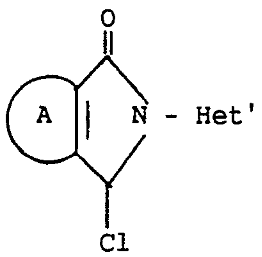 (II) ou 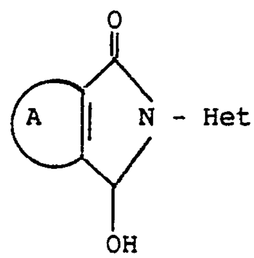 (IV)

dans lesquels A et Het sont définis comme précédemment, et Het' est défini comme précédemment pour Het à l'exception de représenter un radical naphtyridinyle substitué par un radical alcoyloxy sur une amine de formule générale :

R -$NH_2$ (VIII)

dans laquelle R est défini comme précédemment, puis isole le produit obtenu et le transforme, si on le désire et le cas échéant, en un sel pharmaceutiquement acceptable, ou en ce que,

E - Pour la préparation d'un produit de formule générale (I) dans laquelle Y représente un atome d'oxygène, R représente un radical méthyle substitué par un radical alcoylcarbamoyle, dialcoylcarbamoyle, (pipérazinyl-1) carbonyle, pipéridinocarbonyle, et les autres symboles sont définis comme précédemment, on fait agir un composé de formule générale :

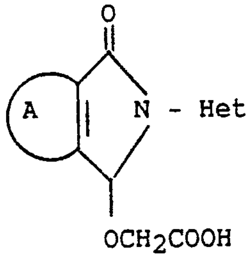

(IX)

ou une forme activée de cet acide,dans laquelle A et Het sont définis comme précédemment sur un

produit de formule générale :

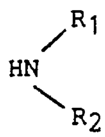

(X)

dans laquelle $R_1$ et $R_2$ représentent chacun un radical alcoyle ou bien $R_1$ représente un atome d'hydrogène et $R_2$ représente un radical alcoyle ou bien $R_1$ et $R_2$ forment avec l'atome d'azote auquel ils sont liés le cycle pipérazine, pipéridine, ces cycles pouvant être substitués comme il est dit précédemment, puis isole le produit obtenu et le transforme, si on le désire, le cas échéant, en un sel pharmaceutiquement acceptable, ou en ce que,

F - Pour la préparation d'un produit de formule générale (I) dans laquelle Y représente un atome d'oxygène et les autres symboles sont définis comme précédemment, on fait agir un produit de formule générale :

ROMe (XII)

dans laquelle R est défini comme précédemment et Me représente un métal alcalin sur un produit de formule générale :

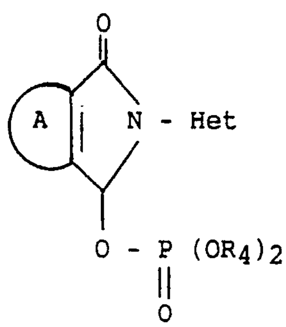

(XIII)

dans laquelle $R_4$ représente un radical alcoyle contenant 1 à 4 atomes de carbone en chaîne droite ou ramifiée ou phényle et A et Het sont définis comme précédemment, puis isole le produit obtenu et le transforme, si on le désire, le cas échéant, en un sel pharmaceutiquement acceptable, ou en ce que,

G - Pour la préparation d'un produit de formule générale (I) dans laquelle Y représente un radical imino et les autres symboles sont définis comme précédemment, on fait réagir un produit de formule générale :

R - X (V)

dans laquelle R est défini comme précédemment et X représente un atome d'halogène ou un reste d'ester réactif, sur un produit de formule générale :

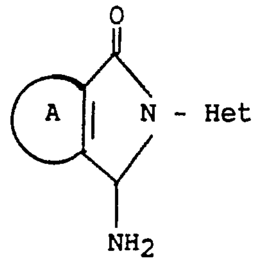

(XV)

dans laquelle A et Het sont définis comme précédemment, puis isole le produit obtenu et le transforme, si on le désire, le cas échéant, en un sel pharmaceutiquement acceptable.

## Claims

**Claims for the following Contracting States : AT, BE, CH, DE, FR, GB, IT, LI, LU, NL, SE**

**1.** New pyrrole derivatives, which are of the general formula:

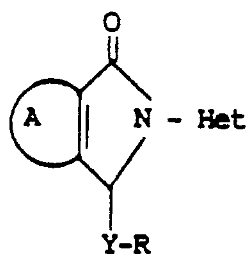

(I)

in which A forms with the pyrrole ring an isoindoline ring-system, Y denotes an oxygen or sulphur atom or an imino radical, Het denotes a naphthyridinyl radical which is substituted with a halogen atom or a (1 to 4 C) alkyl or (1 to 4 C) alkyloxy radical and R denotes an alkyl radical which is unsubstituted or substituted with an alkylcarbonyl, dialkylamino, alkylcarbamoyl, dialkylcarbamoyl, (l-piperazinyl)carbonyl or piperidinocarbonyl radical, on the understanding that the alkyl radicals are straight- or branched-chain radicals and contain, except where specifically stated, 1 to 10 carbon atoms, and that the piperazinyl and piperidino, radicals can be unsubstituted or substituted at any position with an alkyl radical, as well as, when they exist, their pharmaceutically acceptable salts and the optical isomers of the products of formula (I).

**2.** A process for preparing a product according to claim 1 in the formula of which Y denotes an oxygen or sulphur atom, Het is defined as in claim 1 with the exception of denoting a naphthyridinyl radical substituted with an alkyloxy radical, and the other symbols are defined as in claim 1, wherein a product of general formula:

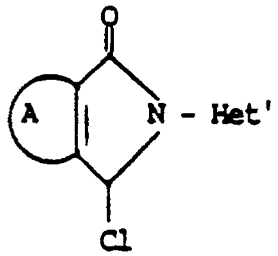

(II)

in which Het' has the definition of Het given in claim 1 with the exception of denoting a naphthyridinyl radical substituted with an alkyloxy radical, and A is defined as in claim 1, is reacted with a product of general formula:

R - Y' - H (III)

in which Y' denotes a sulphur or oxygen atom and R is defined as in claim 1, and the product obtained is then isolated and converted, if so desired, where appropriate, to a pharmaceutically acceptable salt.

**3.** A process for preparing a product according to claim (I) [sic], wherein a product of general formula:

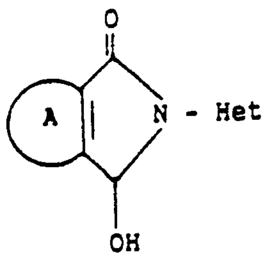

(IV)

in which Het is defined as in claim 1, is reacted with a product of general formula:

R - X (V)

in which R is defined as in claim 1, and X denotes a halogen atom or a reactive ester residue, and the product obtained is then isolated and optionally converted, where appropriate, to a pharmaceutically acceptable salt.

**4.** A process for preparing a product according to claim 1 in the formula of which Y denotes a sulphur atom and the other symbols are defined as in claim 1, wherein a product of general formula:

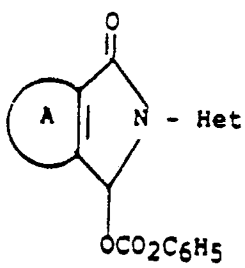

(VI)

in which A and Het are defined as in claim 1, or an equivalent carbonate, is reacted with a mercaptan of general formula:

R - SH (VII)

in which R is defined as in claim 1, and the product obtained is then isolated and converted, if so desired, where appropriate, to a pharmaceutically acceptable salt.

**5.** A process for preparing a product according to claim 1, in the formula of which Y denotes an imino radical and the other symbols are defined as in claim 1, wherein a product of general formula:

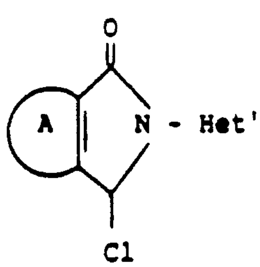

(II) or

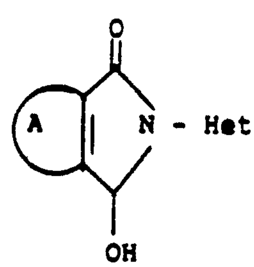

(IV)

in which A and Het are defined as in claim 1, and Het' is defined like Het in claim 1 with the exception of denoting a naphthyridinyl radical substituted with an alkyloxy radical, is reacted with an amine of general formula:

$R - NH_2$ (VIII)

in which R is defined as in claim 1, and the product obtained in then isolated and converted, if so desired and where appropriate, to a pharmaceutically acceptable salt.

6. A process for preparing a product according to claim 1 in the formula of which Y denotes an oxygen atom, R denotes a methyl radical substituted with an alkylcarbamoyl, dialkylcarbamoyl, (1-piperazinyl)-carbonyl or piperidinocarbonyl radical and the other symbols are defined as in claim 1, wherein a compound of general formula:

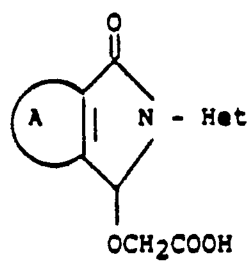

(IX)

in which A and Het are defined as in claim 1, is reacted with a product of general formula:

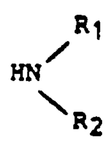

(X)

in which $R_1$ and $R_2$ each denote an alkyl radical, or alternatively $R_1$ denotes a hydrogen atom and $R_2$ denotes an alkyl radical, or alternatively $R_1$ and $R_2$ form, with the nitrogen atom to which they are linked, a piperazine or piperidine ring, it being possible for these rings to be substituted as stated in claim 1, and the product obtained is then isolated and converted, if so desired, where appropriate, to a pharmaceutically acceptable salt.

7. A process for preparing a product according to claim 1 in the formula of which Y denotes an oxygen atom and the other symbols are defined as in claim 1, wherein a product of general formula:

ROMe (XII)

in which R is defined as in claim 1 and Me denotes an alkali metal, is reacted with a product of general formula:

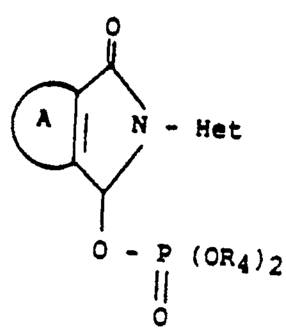

(XIII)

in which $R_4$ denotes a straight- or branched-chain alkyl radical containing 1 to 4 carbon atoms or a phenyl radical, and A and Het are defined as in claim 1, and the product obtained is then isolated and converted, if so desired, where appropriate, to a pharmaceutically acceptable salt.

**8.** A process for preparing a product according to claim 1 in the formula of which Y denotes an imino radical and the other symbols are defined as in claim 1, wherein a product of general formula:

R - X (V)

in which R is defined as in claim 1 and X denotes a halogen atom or a reactive ester residue, is reacted with a product of general formula:

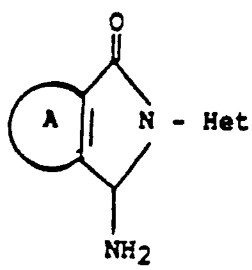

(XV)

in which A and Het are defined as in claim 1, and the product obtained is then isolated and converted, if so desired, where appropriate, to a pharmaceutically acceptable salt.

**9.** Pharmaceutical compositions, which contain at least one product according to claim 1, in combination with one or more diluants or adjuvants that are compatible and pharmaceutically acceptable.

**Claim for the following Contracting States : ES, GR**

**1.** A process for preparing new pyrrole derivatives, of general formula:

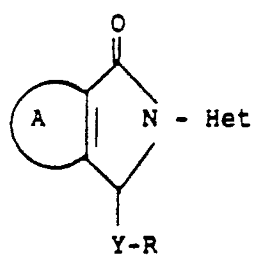

(I)

in which A forms with the pyrrole ring an isoindoline ring-system, Y denotes an oxygen or sulphur atom or an imino radical, Het denotes a naphthyridinyl radical which is substituted with a halogen atom or a (1 to 4 C) alkyl or (1 to 4 C) alkyloxy radical and R denotes an alkyl radical which is unsubstituted or substituted with an alkylcarbonyl, dialkylamino, alkylcarbamoyl, dialkylcarbamoyl, (1-piperazinyl)-carbonyl or piperidinocarbonyl radical, on the understanding that the alkyl radicals are straight- or branched-chain radicals and contain, except where specifically stated, 1 to 10 carbon atoms, and that the piperazinyl and piperidino radicals can be unsubstituted or substituted at any position with an alkyl radical, as well as, when they exist, their pharmaceutically acceptable salts and the optical isomers of the products of formula (I), wherein

A - For the preparation of a product of general formula (I) in which Y denotes an oxygen or sulphur atom, Het is defined as above with the exception of denoting a naphthyridinyl radical substituted with an alkyloxy radical, and the other symbols are defined as above, a product of general formula:

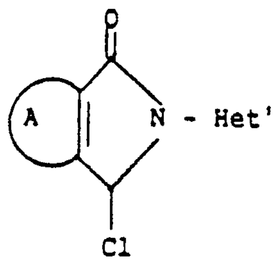

(II)

in which Het' has the definition of Het given above with the exception of denoting a naphthyridinyl radical substituted with an alkyloxy radical, and A is defined as above, is reacted with a product of general formula:

R - Y' - H (III)

in which Y' denotes a sulphur or oxygen atom and R is defined as above, and the product obtained is then isolated and converted, if so desired, where appropriate, to a pharmaceutically acceptable salt, or wherein

B - For the preparation of a product of general formula (I) defined as above, a product of the general formula:

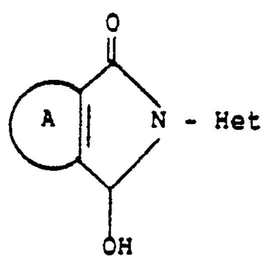

(IV)

in which Het is defined as above, is reacted with a product of general formula:

R - X (V)

in which R is defined as above and X denotes a halogen atom or a reactive ester residue, and the product obtained is then isolated and optionally converted, where appropriate, to a pharmaceutically acceptable salt, or wherein

C - For the preparation of a product of general formula (I) in which Y denotes a sulphur atom and the other symbols are defined as above, a product of general formula:

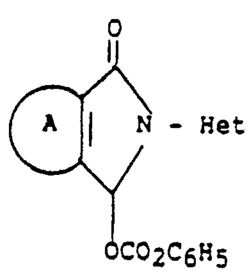

(VI)

in which A and Het are defined as above, or an equivalent carbonate, is reacted with a mercaptan of general formula:

R - SH (VII)

in which R is defined as above, and the product obtained is then isolated and converted, if so desired, where appropriate, to a pharmaceutically acceptable salt,

D - For the preparation of a product of general formula (I) in which Y denotes an imino radical and the other symbols are defined as above, a product of general formula:

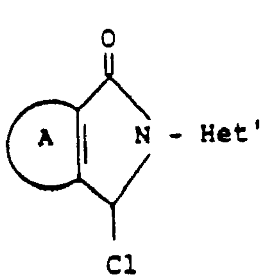 (II) or 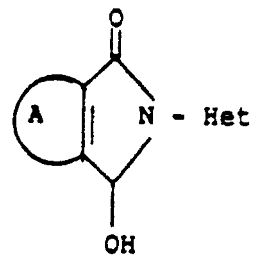 (IV)

in which A and Het are defined as above, and Het' is defined like Het above with the exception of denoting a naphthyridinyl radical substituted with an alkyloxy radical, is reacted with an amine of general formula:

$R - NH_2$ (VIII)

in which R is defined as above, and the product obtained is then isolated and converted, if so desired and where appropriate, to a pharmaceutically acceptable salt, or wherein

E - For the preparation of a product of general formula (I) in which Y denotes an oxygen atom, R denotes a methyl radical substituted with an alkylcarbamoyl, dialkylcarbamoyl, (1-piperazinyl)-carbonyl, or piperidinocarbonyl radical and the other symbols are defined as above, a compound of general formula:

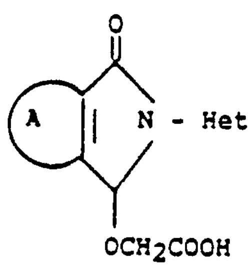

(IX)

or an activated form of this acid, in which A and Het are defined as above, is reacted with a product of general formula:

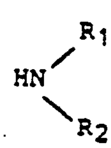

(X)

in which $R_1$ and $R_2$ each denote an alkyl radical, or alternatively $R_1$ denotes a hydrogen atom and $R_2$ denotes an alkyl radical, or alternatively $R_1$ and $R_2$ form, with the nitrogen atom to which they are linked, a piperazine or piperidine ring, it being possible for these rings to be substituted as stated above, and the product obtained is then isolated and converted, if so desired, where appropriate, to a pharmaceutically acceptable salt, or wherein

F - For the preparation of a product of general formula (I) in which Y denotes an oxygen atom and the other symbols are defined as above, a product of general formula:

ROMe (XII)

in which R is defined as above and Me denotes an alkali metal, is reacted with a product of general formula:

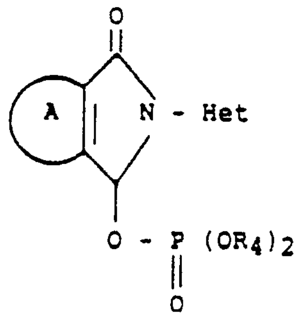

(XIII)

in which $R_4$ denotes a straight- or branched-chain alkyl radical containing 1 to 4 carbon atoms or a phenyl radical, and A and Het are defined as above, and the product obtained is then isolated and converted, if so desired, where appropriate, to a pharmaceutically acceptable salt, or wherein

G - For the preparation of a product of general formula (I) in which Y denotes an imino radical and the other symbols are defined as above, a product of general formula:

R - X (V)

in which R is defined as above and X denotes a halogen atom or a reactive ester residue, is reacted with a product of general formula:

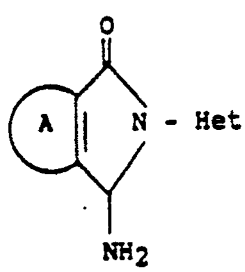

(XV)

in which A and Het are defined as above, and the product obtained is then isolated and converted, if so desired, where appropriate, to a pharmaceutically acceptable salt.

## Patentansprüche

**Patentansprüche für folgende Vertragsstaaten : AT, BE, CH, DE, FR, GB, IT, LI, LU, NL, SE**

**1.** Neue Pyrrolderivate, dadurch gekennzeichnet, daß sie der allgemeinen Formel:

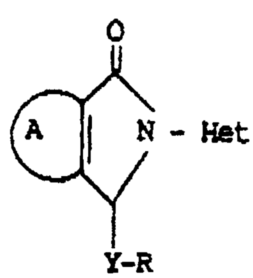

( I )

entsprechen, in welcher A zusammen mit dem Pyrrolring einen Isoindolinkern bildet, Y ein Sauerstoff- oder Schwefelatom oder einen Iminorest darstellt, Het einen durch ein Halogenatom oder einen (1 bis 4 c) Alkyl-, (1 bis 4 C) Alkyloxyrest substituierten Naphthyridinylrest darstellt und R einen gegebenenfalls durch einen Alkylcarbonyl-, Dialkylamino-, Alkylcarbamoyl-, Dialkylcarbamoyl-, (1-Piperazinyl)-carbonyl-, Piperidinocarbonylrest substituierten Alkylrest darstellt, wobei die Alkylreste selbstverständlich geradkettig oder verzweigt sind und ohne spezielle Angabe 1 bis 10 Kohlenstoffatome enthalten und die Piperazinyl-, Piperidinoreste gegebenenfalls in irgendeiner Stellung durch einen Alkylrest substituiert sind, sowie, falls es sie gibt, ihre pharmazeutisch zulässigen Salze und die optischen Isomeren der

Verbindungen der Formel (I).

2. Verfahren zur Herstellung einer Verbindung nach Anspruch 1, in deren Formel Y ein Sauerstoff- oder Schwefelatom darstellt, Het die im Anspruch 1 angegebene Bedeutung mit Ausnahme eines durch einen Alkyloxyrest substituierten Naphthyridinylrestes hat und die übrigen Symbole die im Anspruch 1 angegebene Bedeutung haben, dadurch gekennzeichnet, daß man eine Verbindung der allgemeinen Formel:

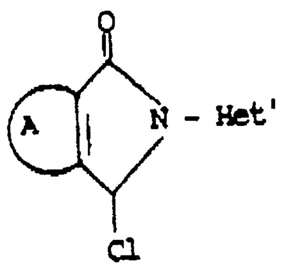

(II)

in welcher Het' die im Anspruch 1 für Het angegebene Bedeutung mit Ausnahme eines durch einen Alkyloxyrest substituierten Naphthyridinylrestes hat und A die im Anspruch 1 angegebene Bedeutung hat, mit einer Verbindung der allgemeinen Formel:

R - Y' - H (III)

in welcher Y' ein Schwefel- oder Sauerstoffatom darstellt und R die im Anspruch 1 angegebene Bedeutung hat, umsetzt, dann die erhaltene Verbindung isoliert und sie gewünschtenfalls zutreffendenfalls in ein pharmazeutisch zulässiges Salz umwandelt.

3. Verfahren zur Herstellung einer Verbindung nach Anspruch 1, dadurch gekennzeichnet, daß man eine Verbindung der allgemeinen Formel

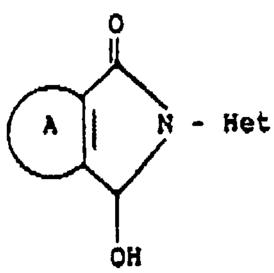

(IV)

in welcher Het die im Anspruch 1 angegebene Bedeutung hat, mit einer Verbindung der allgemeinen Formel:

R - X (V)

umsetzt, in welcher R die im Anspruch 1 angegebene Bedeutung hat und X ein Halogenatom oder einen reaktionsfähigen Ester darstellt, dann die erhaltene Verbindung isoliert und sie gegebenenfalls und zutreffendenfalls in ein pharmazeutisch zulässiges Salz umwandelt.

4. Verfahren zur Herstellung einer Verbindung nach Anspruch 1, in deren Formel Y ein Schwefelatom darstellt und die übrigen Symbole die im Anspruch 1 angegebene Bedeutung haben, dadurch gekennzeichnet, daß man eine Verbindung der allgemeinen Formel

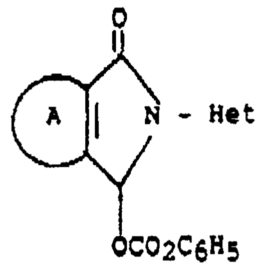

(VI)

in welcher A und Het die im Anspruch 1 angegebene Bedeutung haben, oder ein äquivalentes Carbonat mit einem Mercaptan del allgemeinen Formel:

R - SH (VII)

in welcher R die im Anspruch 1 angegebene Bedeutung hat, umsetzt und dann das erhaltene Produkt isoliert und es gewünschtenfalls zutreffendenfalls in ein pharmazeutisch zulässiges Salz umwandelt.

5. Verfahren zur Herstellung einer Verbindung nach Anspruch 1, in deren Formel Y einen Iminorest darstellt und die übrigen Symbole die im Anspruch 1 angegebene Bedeutung haben, dadurch gekennzeichnet, daß man eine Verbindung der allgemeinen Formel:

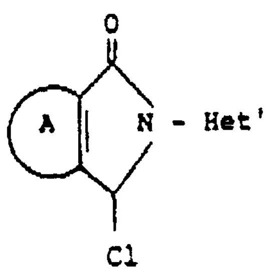

(II) oder

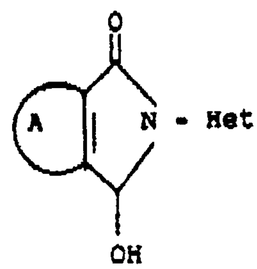

(IV)

in welchen A und Het die im Anspruch 1 angegebene Bedeutung haben und Het' die im Anspruch 1 angegebene Bedeutung mit Ausnahme eines durch einen Alkyloxyrest substituierten Naphthyridinylrestes hat, mit einem Amin der allgemeinen Formel:

R - $NH_2$ (VIII)

in welcher R die im Anspruch 1 angegebene Bedeutung hat, umsetzt, dann das erhaltene Produkt isoliert und es gewünschtenfalls und zutreffendenfalls in ein pharmazeutisch zulässiges Salz umwandelt.

6. Verfahren zur Herstellung einer Verbindung nach Anspruch 1, in deren Formel Y ein Sauerstoffatom darstellt, R einen durch einen Alkylcarbamoyl-, Dialkylcarbamoyl-, (1-Piperazinyl)-carbonyl-, Piperidinocarbonylrest substituierten Methylrest darstellt und die übrigen Symbole die im Anspruch 1 angegebene Bedeutung haben, dadurch gekennzeichnet, daß man eine Verbindung der allgemeinen Formel:

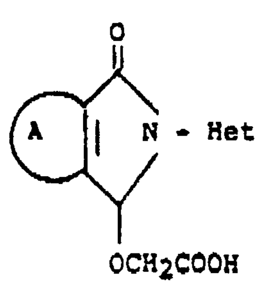

(IX)

in welcher A und Het die im Anspruch 1 angegebene Bedeutung haben, mit einer Verbindung der allgemeinen Formel :

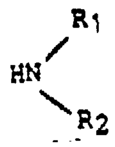

(X)

in welcher $R_1$ und $R_2$ jeweils einen Alkylrest darstellten oder $R_1$ ein Wasserstoffatom darstellt und $R_2$ einen Alkylrest darstellt oder $R_1$ und $R_2$ mit dem Stickstoffatom, an das sie gebunden sind, den Piperazin- oder Piperidinring darstellen, wobei diese Ringe wie im Anspruch 1 angegeben substituiert sein können, dann das erhaltene Produkt isoliert und es gewünschtenfalls und zutreffendenfalls in ein pharmazeutisch zulässiges Salz umwandelt.

**7.** Verfahren zur Herstellung einer Verbindung nach Anspruch 1, in deren Formel Y ein Sauerstoffatom darstellt und die übrigen Symbole die im Anspruch 1 angegebene Bedeutung haben, dadurch gekennzeichnet, daß man eine Verbindung der allgemeinen Formel:

ROMe (XII)

in welcher R die im Anspruch 1 angegebene Bedeutung hat und Me ein Alkalimetall darstellt, mit einer Verbindung der allgemeinen Formel:

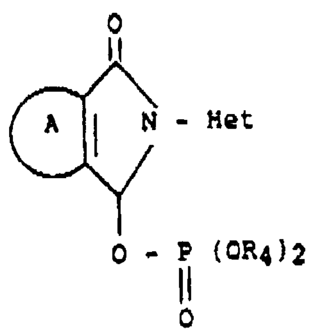

(XIII)

in welcher $R_4$ einen Alkylrest mit 1 bis 4 Kohlenstoffatomen in gerader oder verzweigter Kette oder Phenyl darstellt, und A und Het die im Anspruch 1 angegebene Bedeutung haben, dann die erhaltene Verbindung isoliert und sie gewünschtenfalls zutreffendenfalls in ein pharmazeutisch zulässiges Salz umwandelt.

**8.** Verfahren zur Herstellung einer Verbindung nach Anspruch 1, in deren Formel Y einen Iminorest darstellt und die übrigen Symbole die im Anspruch 1 angegebene Bedeutung haben, dadurch gekennzeichnet, daß man eine Verbindung der allgemeinen Formel

R - X (V)

in welcher R die im Anspruch 1 angegebene Bedeutung hat und X ein Halogenatom oder einen reaktionsfähigen Esterrest darstellt, mit einer Verbindung der allgemeinen Formel:

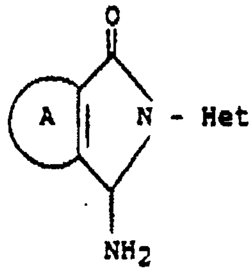

(XV)

in welcher A und riet die im Anspruch 1 angegebene Bedeutung haben, umsetzt, dann die erhaltene Verbindung isoliert und sie gewünschtenfalls und zutreffendenfalls in ein pharmazeutisch zulässiges Salz umwandelt.

9. Pharmazeutische Massen, dadurch gekennzeichnet, daß sie zumindest eine Verbindung nach Anspruch 1 zusammen mit einem oder mehreren verträglichen und pharmazeutisch zulässigen Verdünnungsmitteln oder Zusatzstoffen enthalten.

**Patentanspruche für folgende Vertragsstaaten : ES, GR**

1. Verfahren zur Herstellung neuer Pyrrolderivate der allgemeinen Formel:

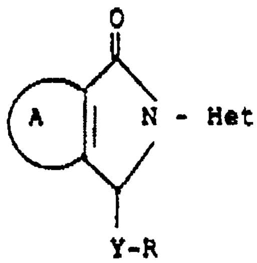

(I)

in welcher A zusammen mit dem Pyrrolring einen Isoindolinkern bildet, Y ein Sauerstoff- oder Schwefelatom oder einen Iminorest darstellt, Het einen durch ein Halogenatom oder einen (1 bis 4 C) Alkyl-, (1 bis 4 c) Alkyloxyrest substituierten Naphthyridinylrest darstellt und R einen gegebenenfalls durch einen Alkylcarbonyl-, Dialkylamino-, Alkylcarbamoyl-, Dialkylcarbamoyl-, (1-Piperazinyl)-carbonyl-, Piperidinocarbonylrest substituierten Alkylrest darstellt, wobei die Alkylreste selbstverständlich geradkettig oder verzweigt sind und ohne spezielle Angabe 1 bis 10 Kohlenstoffatome enthalten und die Piperazinyl-, Piperidinoreste gegebenenfalls in irgendeiner Stellung durch einen Alkylrest substituiert sind, sowie, falls es sie gibt, ihrer pharmazeutisch zulässigen Salze und der optischen Isomeren der Verbindungen der Formel (I),
dadurch gekennzeichnet, daß man

A - zur Herstellung einer Verbindung der allgemeinen Formel (I), in welcher Y ein Sauerstoff- oder Schwefelatom darstellt, Hot die im Anspruch 1 angegebene Bedeutung mit Ausnahme eines durch einen Alkyloxyrest substituierten Naphthyridinylrestes hat und die übrigen Symbole die obige Bedeutung haben, eine Verbindung der allgemeinen Formel:

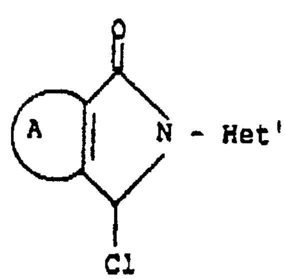

(II)

in welcher Het' die oben für Het angegebene Bedeutung mit Ausnahme eines durch einen Alkyloxyrest substituierten Naphthyridinylrestes hat und A die obige Bedeutung hat, mit einer Verbindung der allgemeinen Formel:

R - Y' - H (III)

in welcher Y' ein Schwefel- oder Sauerstoffatom darstellt und R die obige Bedeutung hat, umsetzt, dann die erhaltene Verbindung isoliert und sie gewünschtenfalls und zutreffendenfalls in ein pharmazeutisch zulässiges Salz umwandelt oder daß man
B - zur Herstellung einer Verbindung der zuvor definierten allgemeinen Formel (I) eine Verbindung der allgemeinen Formel

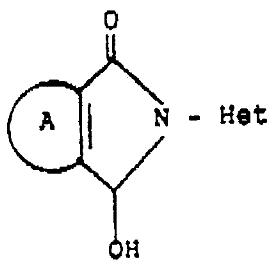

(IV)

in welcher Het die obige Bedeutung hat, mit einer Verbindung der allgemeinen Formel:

R - X (V)

umsetzt, in welcher R die obige Bedeutung hat und X ein Halogenatom oder einen reaktionsfähigen Ester darstellt, dann die erhaltene Verbindung isoliert und sie gegebenenfalls und zutreffendenfalls in ein pharmazeutisch zulässiges Salz umwandelt oder daß man
C - zur Herstellung einer Verbindung der allgemeinen Formel (I) in welcher Y ein Schwefelatom darstellt und die übrigen Symbole die obige Bedeutung haben, eine Verbindung der allgemeinen Formel

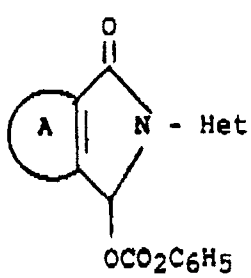

(VI)

in welcher A und Het die obige Bedeutung haben, oder ein äquivalentes Carbonat mit einem Mercaptan der allgemeinen Formel:

R - SH (VII)

in welcher R die obige Bedeutung hat, umsetzt und dann das erhaltene Produkt isoliert und es gewünschtenfalls und zutreffendenfalls in ein pharmazeutisch zulässiges Salz umwandelt,
D - zur Herstellung einer Verbindung der allgemeinen Formel (1), in welcher Y einen Iminorest darstellt und die übrigen Symbole die obige Bedeutung haben, eine Verbindung der allgemeinen Formel:

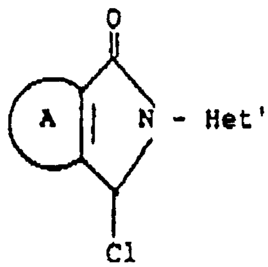 (II) oder 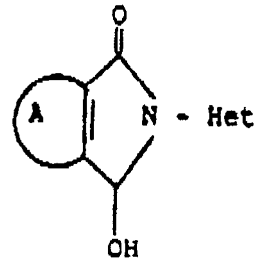 (IV)

in welchen A und Het die obige Bedeutung haben und Het' die obige Bedeutung mit Ausnahme eines durch einen Alkyloxyrest substituierten Naphthyridinylrestes hat, mit einem Amin der allgemeinen Formel:

$R - NH_2$ (VIII)

in welcher R die obige Bedeutung hat, umsetzt, dann das erhaltene Produkt isoliert und es gewünschtenfalls und zutreffendenfalls in ein pharmazeutisch zulässiges Salz umwandelt oder daß man

E - zur Herstellung einer Verbindung der allgemeinen Formel (I), in welcher Y ein Sauerstoffatom darstellt, R einen durch einen Alkylcarbamoyl-, Dialkylcarbamoyl-, (1-Piperazinyl)-carbonyl-, Piperidinocarbonylrest substituierten Methylrest darstellt und die Übrigen Symbole die obige Bedeutung haben, eine Verbindung der allgemeinen Formel:

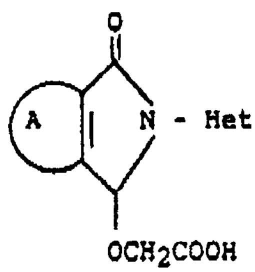 (IX)

oder eine aktivierte Form dieser Säure, worin A und Het die obige Bedeutung haben, mit einer Verbindung der allgemeinen Formel:

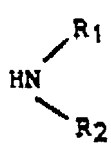 (X)

in welcher R1 und R2 jeweils einen Alkylrest darstellten oder R1 ein Wasserstoffatom darstellt und R2 einen Alkylrest darstellt oder R1 und R2 mit der Stickstoffatom, an das sie gebunden sind, den Piperazin- oder Piperidinring darstellen, wobei diese Ringe wie oben angegeben substituiert sein können, dann das erhaltene Produkt isoliert und es gewünschtenfalls und zutreffendenfalls in ein pharmazeutisch zulässiges Salz umwandelt oder daß man

F - zur Herstellung einer Verbindung der allgemeinen Formel (I), in welcher Y ein Sauerstoffatom darstellt und die übrigen Symbole die obige Bedeutung haben, eine Verbindung der allgemeinen Formel:

ROMe (XII)

in welcher R die obige Bedeutung hat und Me ein Alkalimetall darstellt, mit einer Verbindung der allgemeinen Formel:

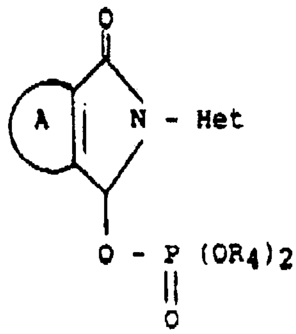

(XIII)

in welcher R4 einen Alkylrest mit 1 bis 4 Kohlenstoffatomen in gerader oder verzweigter Kette oder Phenyl darstellt, und A und Het die obige Bedeutung haben, dann die erhaltene Verbindung isoliert und sie gewünschtenfalls und zutreffendenfalls in ein pharmazeutisch zulässiges Salz umwandelt oder daß man

G - zur Herstellung einer Verbindung der allgemeinen Formel (I), in welcher Y einen Iminorest darstellt und die übrigen Symbole die obige Bedeutung haben, eine Verbindung der allgemeinen Formel

R - X (V)

in welcher R die obige Bedeutung hat und x ein Halogenatom oder einen reaktionsfähigen Esterrest darstellt, mit einer Verbindung der allgemeinen Formel:

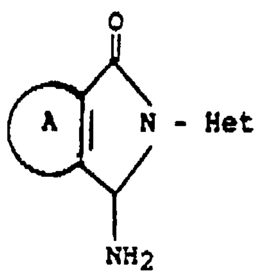

(XV)

in welcher A und Het die obige Bedeutung haben, umsetzt, dann die erhaltene Verbindung isoliert und sie gewünschtenfalls und zutreffendenfalls in ein pharmazeutisch zulässiges Salz umwandelt.